# EUROPEAN PATENT SPECIFICATION

(11) **EP 2 260 097 B1**
(45) Date of publication and mention of the grant of the patent: **26.04.2017**
(21) Application number: 08732046.1
(22) Date of filing: 12.03.2008
(51) Int. Cl.: C12N 5/00

(54) **DISEASE MODEL INCORPORATION INTO AN ARTIFICIAL IMMUNE SYSTEM (AIS)**
EINBEZIEHUNG EINES KRANKHEITSMODELLS IN EIN ARTIFIZIELLES IMMUNSYSTEM (AIS)
INCORPORATION D UN MODÈLE DE MALADIE DANS UN SYSTÈME IMMUNITAIRE ARTIFICIEL (AIS)

(43) Date of publication of application: 15.12.2010
(73) Proprietor: Sanofi Pasteur VaxDesign Corporation, Orlando, FL 32826 (US)
(72) Inventor: WARREN, William L., Orlando, FL 32828 (US); FAHLENKAMP, Heather, Cleveland, OK 74020 (US); HIGBEE, Russell G., Orlando, FL 32825 (US); MISHKIN, Eric M., Winter Springs, FL 32708 (US); SANCHEZ-SCHMITZ, Guzman, Brookline, MA 02446 (US); RIVARD, Michael D., Natick, MA 01760 (US); PAWAR, Santosh, Orlando, FL 32828 (US)
(74) Representative: Owen, Deborah Jane
(86) International application number: PCT/US2008/056720
(87) International publication number: WO 2009/114013

(56) References cited:
- WO-A2-2005/104755
- WO-A2-2007/075979
- WO-A2-2007/106575
- WO-A2-2007/146267
- US-A- 5 008 116
- US-B1- 6 835 550
- HIGBEE RG ET AL: "An immunologic model for rapid vaccine assessment -- a clinical trial in a test tube", ATLA. ALTERNATIVES TO LABORATORY ANIMALS, LONDON, GB, vol. 37, no. Supp. 1, 1 January 2009 (2009-01-01), pages 19-27, XP009142763, ISSN: 0261-1929
- U. MARX ET AL.: 'Drug Testing In Vitro: Breakthroughs and Trends in Cell Culture Technology', 2006, WILEY-VCH. article PORTNER, R ET AL: 'An Overview on Bioreactor Design, Prototyping, and Process Control for ReproducibleThree-Dimensional Tissue Culture', pages 65 - 69, XP008142839
- SUEMATSU ET AL.: 'Generation of a synthetic lyphoid tissue-like organoid in mice' NATURE BIOTECH. vol. 22, no. 12, December 2004, pages 1539 - 1545, XP003013991
- OKAMOTO ET AL.: 'Artificial lymph nodes induce potent secondary immune response in naive and immunodeficient mice' J. CLIN. INVEST. vol. 117, no. 4, April 2007, pages 997 - 1007, XP002623942
- EDELMAN ET AL.: 'A cultureal renaissance: in vitro cell biology embraces three-demensional context' EXP NEUROL. vol. 192, 2005, pages 1 - 6, XP004734474

## Description

### Field of the Invention

The present disclosure is directed to a method for developing a disease model that may be used with an artificial human immune system for *in vitro* testing of vaccines, adjuvants, immunotherapy candidates, cosmetics, drugs, biologics, and other chemicals. The disclosed disease model and artificial immune system is useful for assessing disease pathogenesis and the effect of vaccines, drugs, biologics, immunotherapeutics, and adjuvants on a disease. In the context of vaccines, for example, the disclosed disease models and artificial immune system could be used to predict the effectiveness of a vaccine by means of an *in vitro* challenge with disease agents. The disclosed disease models and artificial immune systems can be prepared using cells from healthy (not diseased, uninfected, naïve) individuals or from individuals suffering from diseases or infections. "Diseased cells" include virally infected cells, bacterially infected cells, and tumor cells, and cells and tissues affected by a pathogen or involved in an immune-mediated disease, such as, *e.g.,* autoimmune disease. Embodiments of the present disclosure can be used to accelerate and improve the accuracy and predictability of vaccine and drug development.

### Background of the Technology

The development and biological testing of human drugs and vaccines has traditionally relied on small animal models (*e.g*., mouse and rabbit models) in the early stages and then on larger animals, such as dogs and non-human primates, in later stages. However, animal models of disease are often only approximations of the human disease state, and in some cases animal models are not available at all *(e.g.,* pathogens that infect only humans). Thus, animal models of disease may not accurately predict outcomes in human studies or may not be available to make such predictions.

In the case of diseases that involve human immunology, such as an immune response to a pathogen or a deleterious inflammatory response, like psoriasis, a major problem remains the translation from animal test systems to human immunology. Successful transfer between traditional testing systems and human biology requires an intricate understanding of disease pathogenesis and immunological responses at all levels. Thus, there is a need for a system that uses human immune cells to simulate human immune responses in the context of a disease state.

### Description of the Invention

The present invention provides artificial immune systems and methods of evaluating *in vitro* the potential reaction of an animal to an agent as defined in the appended claims.

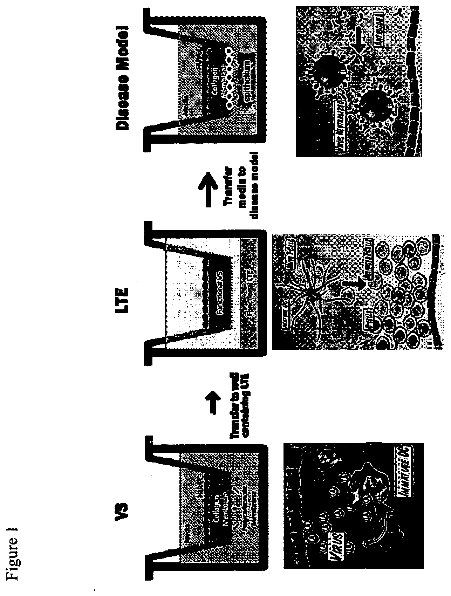

The present disclosure comprises the use of an artificial immune system (AIS) with disease models to provide essentially the ability to conduct a "clinical trial in a test tube" to predict the efficacy of a vaccine, adjuvant, drug, or other agent on a disease that involves the immune system (*e.g*., pathogen response, autoimmune disease, cancer response). Figure 1 illustrates schematically an example of the integration of the AIS and a disease model. As an example, schematically, the VS is where infection or vaccination occurs, the LTE is where immune induction occurs, and the disease model is where the immune response to the disease occurs.

Models of the present disclosure are particularly appropriate for examining diseases and infections that involve immune system cells. Examples include HIV, tuberculosis, tularemia, filoviruses, Yersinia, and Burkholderia. The *in vitro* disease models of the present disclosure can also be used to understand basic disease pathogenesis. The disclosed artificial immune system comprises three modules: the first simulates the innate immune response (the vaccination site, VS, module), the second simulates the adaptive immune response for the detection of T and B cell responses *in vitro,* providing a model for the interaction of immune cells within lymph nodes (the lymphoid tissue equivalent, LTE, module); and the third module is a functional assay or disease module that uses the products of the other two modules together with, for example, a pathogen or human tissue, to measure the effect of the immune response to a disease, such as influenza, glanders, tularemia, tuberculosis, Ebola, Marburg, plague, or AIDS, directly rather than through the use of surrogate markers. These artificial immune system modules reproduce the conditions that exist in the human body, such as the spatial segregation of different immune cells and temporal dynamics that bring different immune cells together at different times. Other variations are possible, such as using the LTE for both immune induction and the immune response, so that the LTE effectively becomes a disease model.

The disclosed disease models and artificial immune systems can be prepared using cells from healthy (not diseased, uninfected, naive) individuals or from individuals suffering from diseases or infections. "Diseased cells" include pathogen-infected cells, *e.g.,* virally infected cells and bacterially infected cells, tumor cells, and cells and tissues affected by a pathogen or involved in an immune-mediated disease, such as, *e.g*., autoimmune disease.

As examples, disclosed disease models include viral (*e.g*., herpes simplex virus, hepatitis A, B, C, VSV, HIV, vaccinia virus, influenza virus), tumoral (*e.g*., melanoma), and autoimmune models (*e.g.,* RA, diabetes, psoriasis, Crohn's disease).

A primary goal of a preclinical testing program is to improve outcome for patients by the early identification of potential applications for new vaccine or drug agents before clinical development. The premise for establishing an *in vitro* testing effort is that it will allow candidates to be selected for clinical evaluation with increased likelihood for clinical benefit. Clearly, this requires that the *in vitro* system be predictive of human responses to the vaccine or drug and the efficacy of the vaccine or drug against the disease in question. In the absence of an effective and predictive preclinical testing program, ineffective vaccines and drugs are likely to be selected for evaluation, thus slowing progress in improving outcomes. Furthermore, having an *in vitro* testing system that is predictive (a "clinical trial in a test tube") will significantly reduce lost opportunity costs associated with vaccine or drug testing. That is, if a candidate is going to fail, it should fail early.

The development of an artificial immune system coupled with a disease model has the potential to change the way vaccines and drugs are tested. The preclinical *in vitro* testing program of the present disclosure, though based on both immunologic and engineering principles, has the very pragmatic objective of providing reliable, predictive, and reproducible information to clinical investigators to allow enlightened prioritization among the multiple candidates available. Clearly, that is a goal of all preclinical testing, but what is new in the *in vitro* testing system of the present disclosure is an *in vitro* model using functionally equivalent tissue engineered constructs populated with human cells. In comparison with *in vivo* animal testing, *in vitro* testing using the system comprising the present disclosure is less expensive, less time-consuming, and importantly more predictive of clinical outcomes.

The present disclosure concerns the development of accurate, predictive *in vitro* models to accelerate vaccine testing, allowing collection of more informative data that will aid in redesigning and optimizing vaccine and drug formulations before animal or clinical trials, and raise the probability that a vaccine or drug candidate will be successful in human trials.

More specifically, the present disclosure comprises controlling the nature and state of the cells in the lymphoid tissue equivalent (LTE, artificial lymph node) of the artificial immune system (AIS). The AIS can be used to test vaccines and other pharmaceuticals for immune reactivity in a manner that is more predictive than animal experiments. Consequently, it can provide valuable pre-clinical data earlier in the research and development process. Antigenic molecules introduced to the AIS are acquired by dendritic cells (DCs) at the vaccination site (VS). The DCs are then transferred to the lymphoid tissue equivalent (LTE), where they present the antigen to T cells, activating their immune function. Activated helper T cells co-stimulate B cells to induce antibody production, while activated cytotoxic T cells lyse antigen-bearing cells. Solubilized antigen(s) can also be introduced into the LTE to directly activate B cells for subsequent antibody production. In other disclosures, pathogens, not antigenic molecules, are introduced to the AIS and infect APCs, *e.g*., dendritic cells (DCs), at the vaccination site (VS). In other disclosures, pathogens are introduced to the AIS and infect cells in the LTE.

While a number of published reports have demonstrated antigen-specific B cell responses (to *C. albicans,* TT, and other antigens) *in vitro,* these results are typically achieved by stimulating and restimulating cultures of whole PBMCs with antigen and exogenous factors to boost B cell proliferation and/or activation. Embodiments of the present disclosure comprise the detection of immune responses using defined cultures of B cells, T cells, and DCs and optionally follicular dendritic cells (FDCs), in 2-dimensional construct assays. The presence of secondary cells provides a more physiological environment for B cell activation and differentiation, such that artificial factors in the cultures are not necessary to detect specific immune responses.

We have generated antigen-specific B cell responses using a (2D) co-culture system comprising T cells, B cells, and antigen-pulsed DCs. Responses have been generated against tetanus toxoid (TT) and a whole protein extract of *Candida albicans (C. albicans)* influenza, recombinant protective antigen of *Bacillus anthracis,* hepatitis B, yellow fever, rabies and merozoite surface protein 1 (MSP-1) from malaria. These results show that culturing human T and B cells together *in vitro* at, for example, a ∼1:1 ratio, versus the ratio of T and B cells naturally found in the blood, gave stronger antigen responses, by both analysis of activation and proliferation (flow cytometry) and antibody production (ELISPOT). Here, "T cells" includes both CD4⁺ and CD8⁺ T cells. In peripheral blood, the T (total T cells):B ratio is ∼7:1. In the lymph node, the T (total T cells):B ratio is ∼1:1.6. In the germinal center, the T:B ratio is ∼1:8, and there, the T cells are primarily CD4⁺ T cells.

It is known that 3-dimensional biology is important to induce proper functionality of immunological engineered tissue constructs (ETCs; see, *e.g.,* Edelman & Keefer, *Exp. Neurol.* **192**:1-6 (2005)). A principal approach to studying cellular processes is to culture cells *in vitro.* Historically, this has involved plating cells on plastic or glass supports. Cells grown on solid or filter support are referred as two-dimensional (2D) cultures. Such 2D cultures on porous supports have been useful in studying many aspects of biology. However, more *in vivo*-like conditions can now be realized in 3D cultures.

In lymph nodes, it has been shown that 3D interstitial tissue matrix facilitates not only T cell migration toward an APC, but also supports motility upon cell-cell interaction. A 3D collagen matrix environment, because of its spatial architecture, provides traction for lymphocyte crawling, mimicking some structural features of the lymph node cortex. This provides experimental justification for the importance of a 3D environment in the constructs that comprise some embodiments of the disclosed artificial immune system.

The present disclosure also differs significantly from existing *in vitro* disease models. For example, simple monolayer and suspension cultures are commonly used to model viral infection and tumors. However, such cell cultures provide a highly artificial cellular environment and are not coupled to an artificial immune system.

The disclosed disease models include cancer models. Although historically mice have been used for studying tumor genetics, physiology, and therapeutic regimens, murine tissue models have many limitations. An important difference is that human tumors are primarily epithelial in origin, whereas murine tumors are typically non-epithelial (such as sarcomas, lymphomas). Many agents that are carcinogenic in mice are not in humans, and *vice versa.* Oncogenic pathways are different in many ways in the mouse compared to humans. Additionally, the murine basal metabolic rate is six times higher than in humans. New approaches have examined xenograft placement on immune- deficient mice with more success; however, the murine component still exists in this model. (Ortiz-Urda et al. (2002) Nature Med 8, 1166-70). Thus, studying human tumor models in a human cell-based model of the present disclosure removes these interspecies differences.

Recent work by Mertsching and colleagues at the Fraunhofer Institute of Interfacial Engineering and Biotechnology, Germany, is beginning to demonstrate that *in vitro* 3D models can be a useful platform in cancer research. They developed a new, 3D, vascularized tissue construct. The vascularized 3D matrix is populated with endothelial cells and then with tumor cells to create an *ex vivo* vascularized tumor-like structure as a disease model. Their data suggests that this *in vitro* model offers the possibility to simulate physiological drug application and provide a human 3D test system for cancer research/therapy.

In the present disclosure, such a 3D tumor model could be used in conjunction with an artificial immune system to predict the effectiveness of a cancer vaccine on a tumor rather than on isolated cancer cells in a 2D culture. This distinction is important. Antibodies and immune effector cells must reach the tumor in sufficient numbers to have an impact on the disease. Access to the cancer cells is not an issue with cell cultures, but can become a problem with a vascularized tumor. In addition, tumors may induce surrounding tissues to secrete factors that induce immune tolerance, thus counteracting the immune enhancement induced by a cancer vaccine. These types of effects may not be observed in the absence of a tumor disease model that interacts with an artificial immune system.

The disclosed disease models include pathogen infection models (*e.g*., viral, bacterial, fungal, protozoan, parasitic). In embodiments of the present invention, we use cells in 2D culture. In other embodiments, we use cells placed with a 3D tissue-engineered construct. The infected or diseased cells can be included in the engineered tissue construct. For example, virally infected epithelial cells can be used in a tissue engineered skin or mucosal equivalent. As another example, herpes simplex viruses are ectodermotropic (*i.e*., they can infect and reproduce in epithelial cells and reside in neurons in a latent state). The disease model in this case could use virally infected epithelial cells or neurons, or both, to model both active and latent herpes virus infection. In other embodiments of the present disclosure, we use engineered tissue constructs to model viral transport and infection in compartments that are separated from the primary infection site.

For example, HIV-1 is captured by dendritic cells (DCs) and delivered to the lymph node, where the virus is then transmitted to CD4⁺ T cells. The lymph node then becomes the principal site of virus production. In an embodiment of the present disclosure, the vaccination site (VS) module would be the infection site, where HIV infects the APCs, e.g., DCs, which would then be placed into the lymphoid tissue equivalent module, where the infected DCs transmit the virus to CD4⁺ T cells. In this case, the LTE would serve as the disease module. Collectively, the infection site and disease module would comprise the disease model.

The disclosed disease models include inflammatory and autoimmune diseases. In these diseases (e.g., psoriasis, rheumatoid arthritis), the immune system itself is primarily responsible for the disease state. In one embodiment of the present disclosure, an LTE could be constructed from immune cells isolated from the blood of donors afflicted with psoriasis. An engineered tissue construct could be made from biopsied skin from the same donor. The interaction of the AIS and the disease model under different conditions could yield insight into disease pathogenesis. Also, one could test different candidate drugs for psoriasis in this disease model to determine potential efficacy of a candidate. If this process was repeated for a large number of donors afflicted with psoriasis, the resulting "clinical trial in a test tube" could facilitate the selection of an optimal clinical candidate for the largest number of patients or the selection of several candidates, which are targeted at different patient populations through the use of clinically relevant biomarkers. In another embodiment of the present disclosure, the disease model would simulate an aspect of an autoimmune or inflammatory disease, rather than the disease itself. For example, an important hallmark of certain inflammatory diseases is the migration of neutrophils to the site of inflammation. If this process of neutrophil migration could be interrupted, then the inflammatory process could be interrupted, with a corresponding beneficial effect on the disease state. The AIS could, thus, be used to model neutrophil migration as a proxy for modeling the resulting inflammatory disease.

HIV models. HIV (human immunodeficiency virus) is the virus that causes AIDS (acquired immune deficiency syndrome). An embodiment of the present disclosure comprises a HIV disease model. *In vivo,* HIV-1 infects DCs and they move to the lymph nodes where the virus infects CD4⁺ T cells. The lymph node then becomes the principal site of virus production.

Infection with HIV-1 is associated with a progressive decrease in the CD4⁺ T cell count and an increase in viral load. The stage of infection can be determined by measuring the patient's CD4⁺ T cell count, and the level of HIV in the blood. This acute viremia is associated in virtually all patients with the activation of CD8⁺ T cells, which kill HIV-infected cells, and subsequently with antibody production, or seroconversion. The CD8⁺ T cell response is thought to be important in controlling virus levels, which peak and then decline, as the CD4⁺ T cell counts rebound to ∼800 cells/mL (normal is ∼1200 cells/mL). A strong CD8⁺ T cell response has been linked to slower disease progression and a better prognosis, though it does not eliminate the virus. During this early phase of infection, HIV is active within lymphoid organs, where large amounts of virus become trapped in the follicular dendritic cells (FDC) network. The surrounding tissues that are rich in CD4⁺ T cells may also become infected, and viral particles accumulate both in infected cells and as free virus.

Tularemia models. *Francisella tularensis* is a Category A biowarfare agent and is an important focus of biodefense research. An embodiment of the present disclosure comprises a tularemia disease model. The pathogenicity of *F. tularensis* is quite similar to that of *Mycobacterium tuberculosis* (Mtb), in that both infect macrophages and dendritic cells (Clemens et al. (2004) Infect. Immun. 72, 3204-17). *F. tularensis* is a Gram-negative bacterium responsible for tularemia, a zoonotic disease that affects many mammals and is occasionally transmitted to humans through tick bites, by direct contact with an infected animal, or through aerosolization of contaminated materials. The progression and the severity of the disease depend on the host immune status and on the infecting strain. There are four known subspecies of *F. tularensis* (*tularensis, holarctica, mediasatica, novicida*); *tularensis* is the most virulent. The live vaccine strain (LVS) was derived from the *holarctica* subspecies and is widely used to study tularaemia. The subspecies *novicida* is less virulent in humans.

*F. tularensis* infects macrophages, dendritic cells, hepatocytes and alveolar epithelial cells. Its virulence depends on its ability to multiply inside host cells. Upon entering the host cell, *F. tularensis* is taken up into a phagosome. It prevents acidification and maturation of the phagosome (Clemens et al. (2004) Infect. Immun. 72, 3204-17), escapes the phagosome, and multiplies in the cytosolic compartment of the host cells. This replication is dependent on a cluster of genes known as the *Francisella* pathogenicity island (FPI). Upon escaping into the cytosol, *F. tularensis* activates many pathways of the innate immune system, including the inflammasome, which triggers both a caspase-1-mediated apoptotic cascade in the host cell and also a pro-inflammatory cytokine response (Henry & Monack (2007) Cell. Microbiol. 9, 2543-255). Cytokines including IL-18, IL-1b, IFN-γ, IL-12, and the Th2 cytokines IL-4 and IL-5, are known to play important roles in the immune response against *F. tularensis* (Henry & Monack (2007) Cell. Microbiol. 9, 2543-255).

Although the role of antibodies in protection against respiratory infection with *F. tularensis* is unclear, one study reported prophylactic and therapeutic use of antibodies for protection against respiratory infection (Kirimanjeswara et al. (2007) J. Immunol. 179, 532-9). Serum antibodies (immune serum from infected mice) were capable of conferring complete protection against lethal respiratory tularemia to a naive mice when given 24-48 h post-exposure.

Filovirus models. The filoviruses Marburg and Ebola are Category A biowarfare agents. An embodiment of the present disclosure comprises a filovirus disease model. Filoviruses are enveloped, non-segmented, negative-stranded RNA viruses. The virions contain a ∼19 kb non-infectious genome that encodes seven structural proteins, with a gene order of: 3' leader, nucleoprotein (NP), virion protein (VP) 35 (VP35), VP40, glycoprotein (GP), VP30, VP24, polymerase L protein, and 5' trailer (Sanchez et al. (199) Virus Res. 29, 215-240). Studies using reconstituted replication systems showed that transcription/replication of Marburg virus requires three of the four proteins (NP, VP35, L), while transcription/replication of Ebola virus requires all four proteins (Muhlberger et al. (1999) J. Virol. 73, 2333-2342). GP is the surface glycoprotein of the virion and is important for receptor binding and membrane fusion (Takada et al. (1997) Proc. Natl. Acad. Sci. USA 94, 14764-69; Ito et al. (1999) J. Virol. 73, 8907-8912).

Most research to date on filovirus infections has come from experimental infection of non-human primates, including cynomolgus macaques and African green monkeys. Rodent models of filovirus infection have provided data on the efficacy of candidate drugs and vaccines, but do not faithfully reproduce the viral pathogenesis and immunity in humans (Bray et al. (2001) J. Comp. Pathol. 125, 243-253; Geisbert et al. (2002) Emerg. Infect. Dis. 8, 503-507). Indeed, there is currently only limited data available on the pathophysiology of filovirus infections in humans.

Both Ebola and Marburg viruses have broad cell tropisms and use a variety of host cell surface molecules to gain entry into host cells. Infection of monocytes/macrophages and dendritic cells is central to the pathology of Ebola virus infection (Stroher et al. (2001) J. Virol. 75, 11025-33), triggering a cascade of events leading to the production and release of the procoagulant protein tissue factor (TF) (Geisbert et al. (2003a) J. Infect. Dis. 188, 1618-1629) and a variety of cytokines/chemokines (Stroher et al. (2001) J. Virol. 75, 11025-33; Hensley et al. (2002) Immunol. Lett, 2002, 80, 169-179). Although lymphocytes are not infected by Ebola or Marburg viruses, there is large-scale destruction of these cells, said to be the result of "bystander" apoptosis (Geisbert et al. (2000) Lab. Invest. 80, 171-86). Reasons for this aberrant apoptosis of lymphocytes are unclear. An embodiment of the present disclosure comprises an *in vitro* disease model that can be used to explore this phenomenon.

It has been suggested that infection of monocytes/macrophages, damage to endothelial cells, and release of procoagulant protein tissue factor appear to be the cause for the development of hemorrhage, shock, and coagulation defects such as disseminated intravascular coagulation (DIC) during filoviral infections (Geisbert et al. (2003a) J. Infect. Dis. 188, 1618-1629).

In studies involving infection of non-human primates with Ebola virus, increased circulating levels of IFNa□, IL-6, MCP-1, MIP-1a, MIP-1b, IFN-β, IFN-γ, IL-18, and TNF-α □at different stages of disease were observed (Geisbert et al. (2003b) Am. J. Pathol. 163, 2347-2370). In human cases, an association between increased levels of IL-10 and increased fatalities was observed in Ebola virus infection (Baize et al. (2002) Clin. Exp. Immunol. 128,163-168). Other *in vitro* studies with different types of primary human cells showed similar increases in proinflammatory cytokine levels (Stroher et al. (2001) J. Virol. 75, 11025-33; Hensley 2002) and also that results varied due to human donor-associated genetic differences. In using the VS module to model filovirus disease, cytokine production in the VS module is monitored as are phenotypic changes in the APCs.

Early attempts to develop filoviral vaccines have used cell culture-propagated filoviruses inactivated with formalin or heat treatment, but the protection offered was inadequate (Geisbert et al. (2002) Emerg. Infect. Dis. 8, 503-507). Current efforts use different recombinant vectors, such as VSV and adenoviral vectors, for expression of filoviral-encoded proteins individually or in combinations (Geisbert & Jahrling (2003c) Exp. Rev. Vaccines 2, 777-789). GP and NP are being tested as vaccine candidates in non-human primates with encouraging results (Sullivan et al. (2003) Nature 424, 681-4). There are currently no effective post-exposure treatments for filoviral infections.

*Yersinia models*. The genus *Yersinia* includes three species that are pathogenic to humans, *Y. enterocolitica, Y. pestis,* and *Y. pseudotuberculosis* (Brubaker (1991) Clin. Microbiol. Rev. 4, 309-324). An embodiment of the present disclosure comprises a *Yersinia* disease model. *Y. pestis,* a Gram-negative bacterium, is the agent of plague, a lethal disease transmitted by flea bites or by aerosols (Perry & Fetherston (1997) Clin. Microbiol. Rev. 10, 35-66). *Y. pestis* has been the cause of three pandemics (Drancourt et al. (2004) Emerg. Infect. Dis. 10, 1585-92), and has resulted in the deaths of millions of people.

*Y. pseudotuberculosis* and *Y. pestis* are closely related. It is believed that *Y. pestis* may have evolved from *Y. pseudotuberculosis* (Skurnik et al. (2000) Mol. Microbiol. 37, 316-330; Achtman et al. (1999) Proc. Natl. Acad. Sci. USA 96, 14043-48). Minor phenotypic differences have been used to classify *Y. pestis* strains into three biovars (Antiqua, Mediaevalis, Orientalis) (Perry & Fetherston (1997) Clin. Microbiol. Rev. 10, 35-66). Despite differences in their mode of entry into the host and severity of disease, all three pathogenic *Yersinia* species exhibit a common tropism for lymphoid tissue (Brubaker (1991) Clin. Microbiol. Rev. 4, 309-324). The complete genome sequence of *Y. pestis* has been determined (Parkhill et al. (2001) Nature 413, 523-527; Deng W et. al. (2002) J. Bacteriol. 184, 4601-4611).

In the pathogenic *Yersinia* spp., several virulence factors have been identified that promote serum resistance and the acquisition of iron (Brubaker (1991) Clin. Microbiol. Rev. 4, 309-324; Perry & Fetherston (1997) Clin. Microbiol. Rev. 10, 35-66; Camiel 2002). Additionally, they contain a 70-kb plasmid that is necessary for sustained bacterial replication in host tissues (Cornelis et al. (1998) Microbiol. Mol. Biol. Rev. 62, 1315-1352). A type III secretion system (TTSS) and several secretion substrates (Yops, LcrV) are expressed from the virulence plasmid when *Yersinia* spp. are grown at 37°C (Cornelis et al. (1998) Microbiol. Mol. Biol. Rev. 62, 1315-1352; Perry & Fetherston (1997) Clin. Microbiol. Rev. 10, 35-66). After they are secreted by the TTSS, the Yops are delivered into the phagocytes, where they inhibit phagocytosis and proinflammatory cytokine production and also trigger apoptosis of host cells (Cornelis (2002) J. Cell. Biol. 158, 401-408). LcrV is secreted into the extracellular milieu where it inhibits inflammation by interacting with Toll-like receptor 2 (Brubaker (2003) Infect. Immun. 71, 3673-3681). *Y. pestis* carries two plasmids, pMT1 and pPCP1, that impart *Y. pestis* with increased virulence (pMT1 and pPCP1) and vector-borne transmissibility (pMT1) (Carniel (2002) Curr. Top. Microbiol. Immunol. 264, 89-108; Perry & Fetherston (1997) Clin. Microbiol. Rev. 10, 35-66).

*Y. pestis* causes *bubonic* plague when its mode of entry is intradermal following the bite from an infected flea, while it causes *pneumonic* plague when infection is by inhalation of infectious droplets (Brubaker (1991) Clin. Microbiol. Rev. 4, 309-324; Perry & Fetherston (1997) Clin. Microbiol. Rev. 10, 35-66). Disease pathogenesis has been studied in mice and non-human primates (Welkos et al. (1997) Microb. Pathogen. 23, 211-223; Finegold (1969) Am. J. Pathol. 54, 167-185). Bacteria multiply at the initial site of infection before entering the lymphatic system and spread to regional lymph nodes and via the bloodstream to other organs, such as spleen and liver. Macrophages may act as the vehicle for transport from the initial site of infection to the lymphoid tissues. Extensive bacterial replication in visceral organs leads to septicemia and death of the host (Brubaker (1991) Clin. Microbiol. Rev. 4, 309-324; Perry & Fetherston (1997) Clin. Microbiol. Rev. 10, 35-66). Animal studies have indicated that in the later stages of infection process (more than 12 h post-infection) *Y. pestis* was found to replicate in necrotic foci extracellularly (Welkos et al. (1997) Microb. Pathogen. 23, 211-223; Nakajima et al. (1995) Infect. Immun. 63, 3021-3029).

*Y. pestis* has long been considered a facultative intracellular pathogen (Cavanaugh & Randall (1959) J. Immunol. 85, 348-363). Animal studies have indicated that *Y. pestis* can survive and replicate within macrophages (Finegold 1969), but is killed intracellularly by neutrophils (Cavanaugh & Randall (1959) J. Immunol. 85, 348-363; Burrows & Bacon (1956) Br. J. Exp. Pathol. 37, 481-493). Thus, macrophages effectively serve as permissive sites for replication in the early stages of infection. *Y. pestis* achieves this by subverting the normal antibacterial functions of macrophages. A study in inbred and outbred strains of mice infected with pneumonic plague showed that proinflammatory cytokines, such as like IL-6, TNFα, IFNγ, IL-12, and MCP-1 are found in the bronchoalveolar lavage fluids in later stages of infection (Bubeck et al. (2007) Infect. Immun. 75, 697-705). In the host immune response to plague, *Y. pestis*-infected human monocytes were reported to express TLR9 and differentiate into dendritic cells (Saikh et al. (2004) J. Immunol. 173, 7426-34). *Y. pestis* is known to evade immune responses in part by injecting host immune cells with several effector proteins called *Yersinia* outer proteins (Yops) that impair cellular function. *Y. pestis* YopJ disrupts signal transduction pathways and interferes with DC differentiation and subsequent function (Lindner et al. (2007) Eur. J. Immunol. 37, 2450-62). Further, YopJ injection prevents upregulation of costimulatory ligands, and LPS-induced cytokine expression in DC thus crippling the adaptive response via a diminished capacity to induce T cell proliferation and IFNγ induction.

An effective vaccine should induce both humoral and cellular immune responses that contribute to protection (Zinkernagel (2003) Annu. Rev. Immunol. 21, 515-546). Humoral immunity involves antibody production by B cells that act to neutralize an extracellular pathogen, its proteins, and toxins, while cellular immunity involves production of cytokines and cytolytic capacities of T cells and acts to eradicate intracellular pathogens. Vaccines composed of either killed pathogen or purified proteins mixed with adjuvants act by priming humoral immunity (Meyer et al. (1974) J. Infect. Dis. 129 (Suppl.), S13-S18; Heath et al. (1998) Vaccine 16, 1131-1137). In contrast, live attenuated vaccines of virulent pathogens, act by priming cellular immunity (Levine & Sztein (2004) Nat. Immunol. 5, 460-464). The importance of cellular immunity in providing vaccine protection against *Y. pestis* has been demonstrated using a mouse model (Parent et al. (2005) Infect. Immun. 73, 7304-10). This report shows the importance of CD4 and CD8 T cells in immunity to *Y. pestis* and that IFNγ and TNFα secreted by these cells played an important role in it.

Early plague vaccine research centered on the bubonic form of disease. Heat-killed cultures of virulent *Y. pestis* formulated as vaccine were used by Haffkine in 1897 (Haffkine (1897) Br. Med. J. 1, 1461). Kolle & Otto found that live attenuated *Y. pestis* strains protected mice against virulent infection (Kolle & Otto (1904) Z. F. Hyg. 48, 399-428). Though these live attenuated strains were used in humans and their safety and efficacy was established (Strong (1908) J. Med. Res. 18, 325-346), they occasionally caused adverse reactions. Pneumonic plague vaccine efforts have largely focused on the development of subunit vaccines using recombinant *Y. pestis* proteins (Titball & Williamson (2004) Expert Opin. Biol. Ther. 4, 965-973); fraction 1 (F1) and V have been widely tested in vaccinations and these recombinant proteins protects mice against pneumonic plague (Williamson et al. (1995) FEMS Immunol. Med. Microbiol. 12, 223-230; Anderson et al. (1996) Infect. Immun. 64, 4580-4585; Andrews et al. (1996) Infect. Immun. 64, 2180-2187). A recombinant F1-V fusion protein vaccine has been reported to protect mice (Heath et al. (1998) Vaccine 16, 1131-1137), but does not fully protect non-human primates against pneumonic plague.

The current treatment regimen for plague comprises use of the antibiotics tetracycline, streptomycin, and chloramphenicol. Recently gentamicin, chloramphenicol, doxycycline and ciprofloxacin have also been recommended.

Burkholderia models. *Burkholderia mallei,* is a category B biowarfare agent per the CDC classification. *B. mallei* is a Gram-negative, non-motile bacillus, and causes glanders primarily in horses, mules, and donkeys. An embodiment of the present disclosure comprises a burkholderia disease model. It infects by the oral route and spread is by close contact with infected animals. Infection of horses is most often manifested as a slow progressive, chronic disease, whereas in donkeys, the disease is usually severe, causing death in 7-10 days (Acha & Szyfres (1987) Zoonoses and communicable diseases common to man and animals. 2nd ed. Washington, DC: World Health Organization). Other animals such as mice, hamsters, guinea pigs, monkeys, and dogs, are also susceptible to this pathogen (DeShazer 2004). *B. mallei* can also infect by the cutaneous route. There is no effective treatment for glanders in the natural host, and animals diagnosed with glanders are typically isolated and destroyed. In humans, infection with *B. mallei* can occur via mucosal (oral, nasal, ocular) or cutaneous routes. Currently, there is no vaccine against *B. mallei.* Because of its potential use as a biowarfare agent, a vaccine against *B. mallei* is a high priority. Indeed, *B. mallei* was used as a bioweapon in the First World War by German troops to disable the Russian army's horses and mules (Aldhous (2005) Nature 434, 692-3).

A genetically related species, *Burkholderia pseudomallei,* causes melioidosis which has a fatality rate of almost 50% in countries such as Thailand (Aldhous (2005) Nature 434, 692-3). It is endemic in parts of Asia and in northern Australia. *B. pseudomallei* is also considered a potiential bioterrorism agent. Another species, *Burkholderia thailandensis,* is considered non-pathogenic in humans.

The complete genome sequence of *B. pseudomallei* (Holden et al. (2004) Proc. Natl. Acad. Sci. USA 101, 14240-14245) has been determined by the Wellcome Trust, UK, and that of *B. mallei* (Nierman et al. (2004) Proc. Natl. Acad. Sci. USA 101, 14246-51) was determined by TIGR. It appears that *B. mallei* evolved from *B. pseudomallei* by deletion of portions of its genome (Godoy et al. (2003) J. Clin. Microbiol. 41, 2068-2079). There are reports that gene losses have contributed to the pathogenic evolution of bacterial species (Maurelli et al. (1998) Proc. Natl. Acad. Sci. USA 95, 3943-3948; Moore et al. (2004) Infect. Immun. 72, 4172-87). The non-pathogenic *B. thailandensis* has the ability to assimilate arabinose using proteins encoded by an arabinose assimilation operon, while the pathogenic species *B. mallei* and *B. pseudomallei* have lost this operon. Thus, genes for arabinose assimilation have been termed anti-virulence genes (Moore et al. (2004) Infect. Immun. 72, 4172-87).

Analysis of the genome sequence of *B. pseudomallei* identified several genes encoding survival and virulence functions, including three type III secretion system (TTSS) genes, while in *B. mallei* genes responsible for the virulence function form a gene cluster encoding an exopolysaccharide capsule (DeShazer et al. (2001) Microb. Pathogen. 30, 253-269) and a TTSS (Ulrich & DeShazer (2004) Infect. Immun. 72, 1150-1154). The TTSS was found to be essential for intracellular survival of *Burkholderia mallei* within human macrophage-like cells (Ribot & Ulrich (2006) Infect. Immun. 74, 4349-53).

Several animal species have been used a models of human *B. mallei* infection, including monkeys, guinea pigs, hamsters, and mice. Acute glanders in humans is characterized by rapid onset of pneumonia; as a result, an aerosol model of infection in Balb/c mice was developed (Lever et al. (2003) J. Med. Microbiol. 52, 1109-15). In the initial stage of the disease, the pathogen localizes in the upper and lower sections of the respiratory tract and is transported by alveolar macrophages to regional lymph nodes. As disease progresses, bacteria disseminate and are also found in other organs, including liver and spleen, and in the bloodstream in later stages.

In the host immune response to *B. mallei* infection, type I cytokines, IFNγ and IL-12, are key in controlling the initial infection (Rowland et al. (2006) Infect. Immun. 74, 5333-40). In that report, increased levels of IFNγ, IL-6, MCP-1, IL-12p35, IL-18, and IL-27 were found in the serum and spleen of peritoneally-infected mice. IFNγ knockout mice were unable to control infection and died within 2-3 days, suggesting the importance of IFNγ in host immunity to *B. mallei* infection. *B. mallei* has an outer lipopolysaccharide (LPS) capsule. It has been reported that *B. mallei* LPS is a potent activator of human Toll-like receptor (TLR) 4 (Brett et al. (2007) Mol. Microbiol. 63, 379-90), eliciting TLR4-mediated stimulation of human macrophage-like cells (THP-1, U-937), monocyte-derived macrophages, and dendritic cells, resulting in high levels of TNF-α, IL-6, and RANTES. These observations suggest that the *B. mallei* LPS capsule plays an important role in the pathogenesis of the human disease.

As *B. mallei* and *B. pseudomallei* are naturally soil-dwelling microbes, they are intrinsically resistant to many antibiotics. In a study on 65 isolates of *B. mallei* and *B. pseudomallei,* a wide range of resistance to antimicrobial agents was noted, including to fluoroquinolones, β-lactam antibiotics, aminoglycosides, and macrolides. Bacteria were found to be susceptible to imipenem, ceftazidime, piperacillin, piperacillin / tazobactam, doxycycline, and minocycline (Thibault et al. (2004) J. Antimicrob. Chemother. 54, 1134-8). These antibiotics are currently being used in post-exposure treatment of melioidosis and glanders.

A report indicated that pretreatment of Balb/c mice with an oligodeoxynucleotide (ODN) containing CpG motifs (CpG ODN 7909) protected them against aerosol challenge with *B. mallei.* This protection was found to be associated with enhanced levels of interferon gamma (IFNγ)-inducible protein 10 (IP-10), IL-12, IFNγ, and IL-6. Thus, treatment with CpG ODN 7909 provided an effective pre-exposure therapy to protect against glanders. CpG ODN 7909 is an agonist of TLR9. TLRs 7 and 9 are thought to share the MyD88-dependent pathway that activates interleukin-1 receptor-associated kinases (IRAKs) and TRAF6 located downstream (Kawai & Akira (2005) Arthritis Res. Ther. 7, 12-19). These, in turn, activate NF-κB and mitogen-activated protein (MAP) kinases, leading to activation of inflammatory cytokine genes. Thus, TLR agonists activating TLR7/9 or other TLRs may act to protect against lethal infection with glanders.

In summary, in the diseases described above, the pathogenesis of HIV, Yersinia, Burkholderia, filoviruses, and tularemia all involve antigen-presenting cells being infected with a pathogen, which they then transport to regional lymph nodes where pathogen replication occurs. As an example, the *in vitro* infectious disease model can be comprised of two modules: an infection module, where the macrophages/DCs are infected by the pathogen, which can be represented in the disclosed model systems by the VS, and the disease module, which represents the process whereby infected APCs transport the pathogen to regional lymph nodes, which can be represented in the disclosed model systems by the LTE. In this disease model module LTE, the lymphocytes are activated/primed and the pathogen multiplies, as occurs naturally in a human lymph node. For this category of pathogen, the LTE itself may serve as the disease model module rather than needing a separate engineered tissue construct as with, *e.g.,* a tumor disease model. Optionally, one could use the AIS to generate an immune response to a potential vaccine candidate and then transfer the contents of the non-infected, primed LTE to an infected LTE disease model module to determine the effect of the vaccine candidate on viral replication and infection, and the effect on immune clearance of the infection. Because the present disclosure is modular, with both infected and uninfected VS and LTE modules, and the modules can be used in different combinations and sequences, a practioner may select different embodiments to investigate different effects on viral replication, infection, and pathogenesis resulting from different antigens, adjuvants, vaccines, drugs, and other agents, depending on whether the effect sought is prophylactic, therapeutic, or both, and depending on the desired site of immunity (e.g., peripheral tissue, lymphoid tissue, or both). Indeed, some embodiments of the disclosure may use only a modified VS or LTE as the entire disease model for certain types of disease.

### Brief Description of the Figures

Figure 1. Schematic illustration of an embodiment of the disclosure, showing the integration of the AIS and a disease model.
Figure 2. A 3D heterogeneous tissue construct, comprising the addition of cells on the top and bottom of the construct, to create endothelial and epithelial layers.
Figure 3. A schematic representation of the development of a generic disease model and how it can be tested with a particular disease.
Figure 4. Schematic illustration of, as an example, vertically expanding melanoma tumor cells or bacterially or virally infected fibroblast cells inside the 3D construct.
Figure 5. Flow chart for preparation and analysis of an *in vitro* tularemia model.

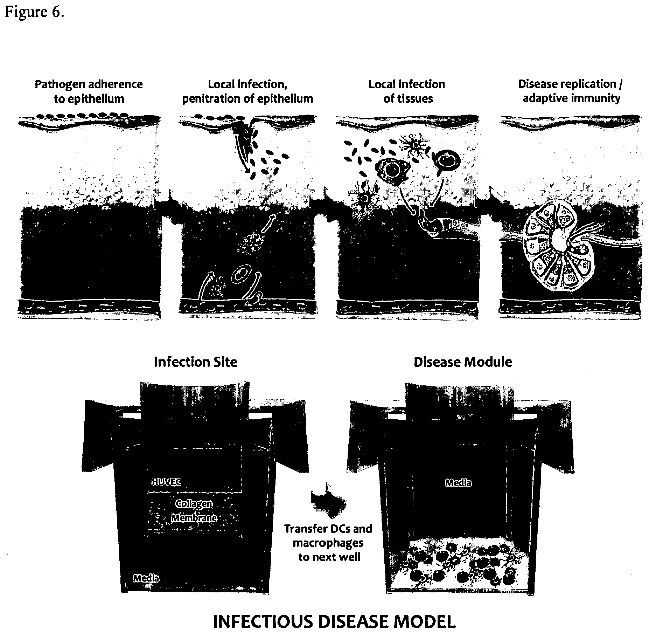

Figure 6. An example *in vitro* disease model architecture.

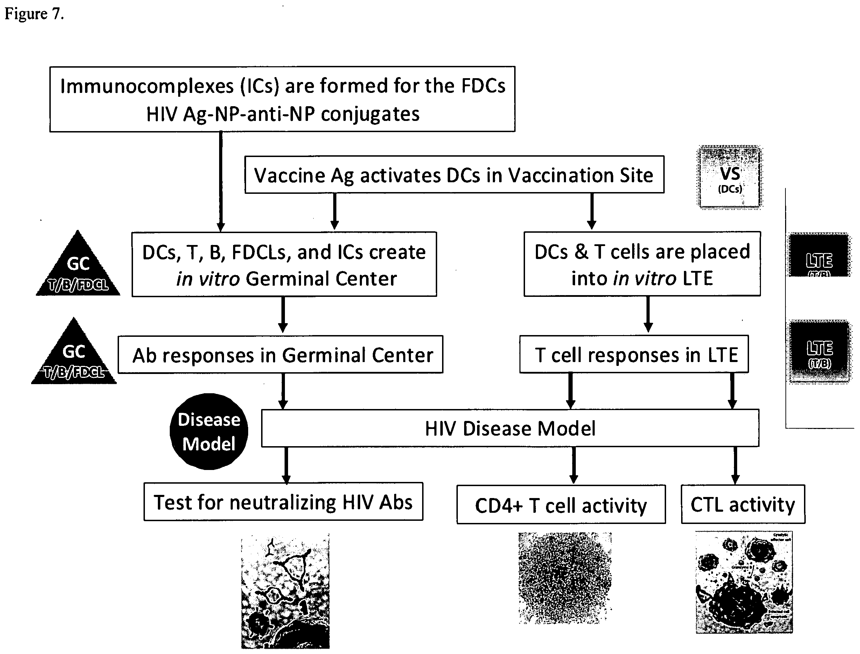

Figure 7. HIV vaccine candidate testing flow chart.

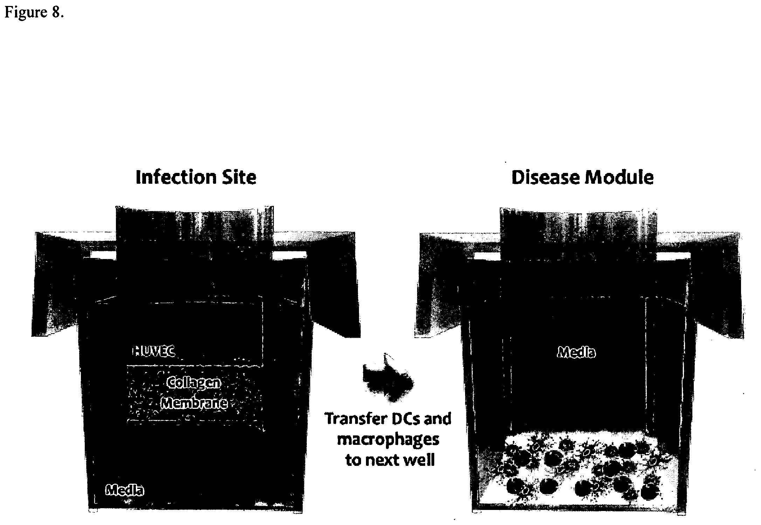

Figure 8. HIV disease model for testing HIV vaccine candidates. A candidate vaccine is placed in the VS module to prime APCs. These APCs are then placed in the LTE module to prime lymphocytes. The *in vitro*-immunized lymphocytes and resulting antibodies are placed in the disease module along with infected APCs (obtained from a parallel infection with HIV in the infection site module). Autologous cells are used. Any protection offered is quantitated, for example, by inhibition of viral replication or cell lysis.

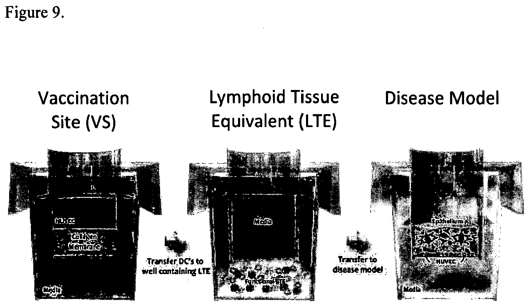

Figure 9. Assessment of correlates of protection in an infectious disease. All elements of correlates of protection (CTL, antibodies, cytokines, CD4⁺ T cells) are examined.

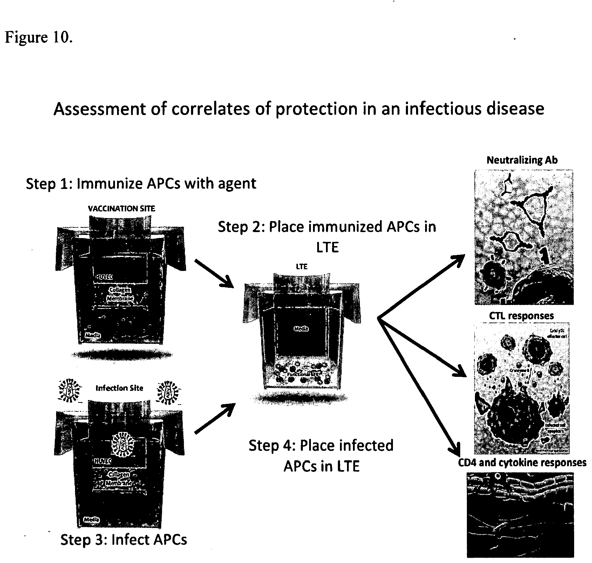

Figure 10. Diseased cells (e.g., HIV-infected APCs) would be exposed to 'output' from the VS/LTE combination by putting the diseased cells (e.g., HIV-infected APCs) from the VS into the LTE to evaluate the effect on the diseased cells. The effects on the LTE components by the diseased cells can be assessed, as can any effect on the diseased cells to define the correlates of protection.

### Examples

The artificial immune system of the present invention can be used to study many human diseases, including HIV. This *in vitro* model can not only be used to model bacterial and viral disease systems, but also used to study host immune responses in injury and inflammation.

### Example 1

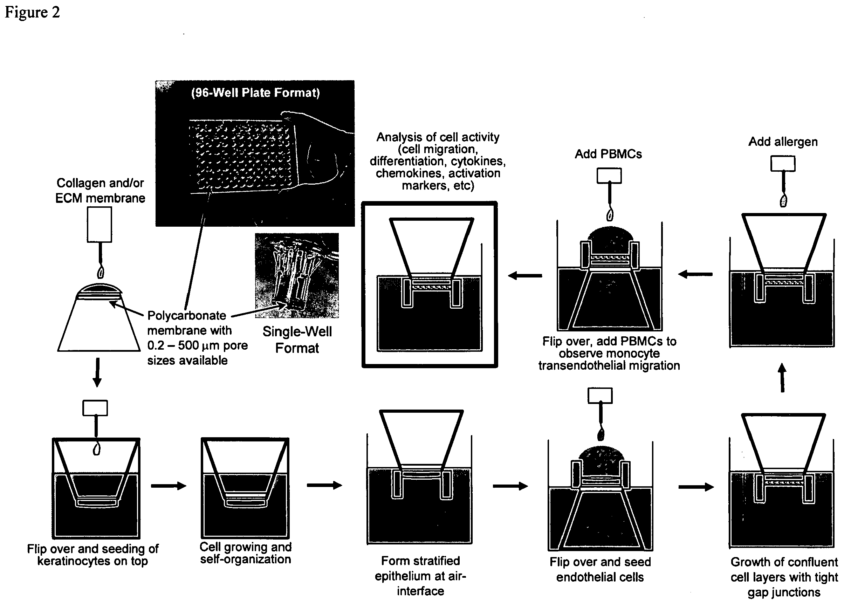

Generic tissue construct for a 3D *in vitro* disease model. Figure 2 illustrates a 3D heterogeneous tissue construct, comprising the addition of cells on the top and bottom of the construct, to create endothelial and epithelial layers. This model is an improvement on our established 3D endothelial cell-only construct, which has been used for transendothelial migration and for monocyte to dendritic cell and macrophage differentiation (the vaccination site, VS).

The 3D model of this example can be used to study immunophysiological reactions when subjected to various diseases and vaccine formulations. This is a generic construct because most tissues involve a 3D extracellular matrix with associated endothelial and epithelial layers. The disease, whether viral, bacterial, or tumoral, is introduced into the generic tissue construct. The various immunocytes and biomolecules from the AIS (*e.g.,* antibodies, T cells, cytokines, chemokines) can then be delivered to the disease model to examine and detect effector responses (*e.g*., the presence or absence of neutralizing antibodies, cytotoxicity).

### Example 2.

Tumor modeling in the AIS using melanoma cells. Many *in vitro* model systems have been used for examining the effects of anti-cancer therapeutics and tumor growth in adult and childhood cancers, using both primary cells and various cell lines (*see, e.g.,* Houghton et al. (2002) Clin Cancer Res 8, 3646-57). Such models have proven useful for assessing tumor metabolic states, inhibition of proliferation, and decreases in overall biomass (*see, e.g.,* Monks et al., (1991) J Natl Cancer Inst 83, 757-66; Scherf et al., (2000) Nat Genet 24, 236-44).

Animal models of human cancers have not been good predictors of human therapeutic outcome because of species differences (*see, e.g.,* Houghton et al.,(2002) Clin Cancer Res 8, 3646-57; Bridgeman et al.,(2002) Cancer Res 60, 6573-6; Batova et al.,(1999),Cancer Res 59, 1492-7).

As with any tumor model, the primary end goal is to increase patient survival and overall well being and to decrease tumor burden. The most predictive model will aid in correlating between what is observed *in vitro* with what is observed in the clinical setting.

Melanocytes in human skin are inter-follicular melanin-containing (pigmented) cells within the epithelial stratum and are of neuroectodermal origin. Melanoma is a common form of human skin cancer. Malignant melanoma (both pigmented and non-pigmented forms) are frequently resistant to interventional therapies and are associated with significant morbidity and mortality.

Two modes of melanoma cellular proliferation are known to occur: one in a radial direction and the other in a vertical direction, into the subepithelial matrix (dermal layer *in vivo*) (Chudnovsky et al., (2005) Nat Genet 37, 745-9). Many factors have been implicated in spontaneous, uncontrolled proliferation including genetic alterations, overexpression of the catalytic subunit of human telomerase reverse transcriptase (TERT) and expression of melanoma markers HMB-45 and Melan-A. Pagetoid invasion into upper epithelial and dermis layers is typically observed under these conditions. Various melanoma cells can be purchased from ATCC *(e.g.,* A-375, SK-Mel-31, WM115, SK-Mel-2, SK-Mel-24) with varying characteristics as to invasion properties (vertical or radial) and expression of specific human melanoma markers (*e.g*., NRAS, PI3K, CDK4, HMB-45 or Melan-A).

### Example 3.

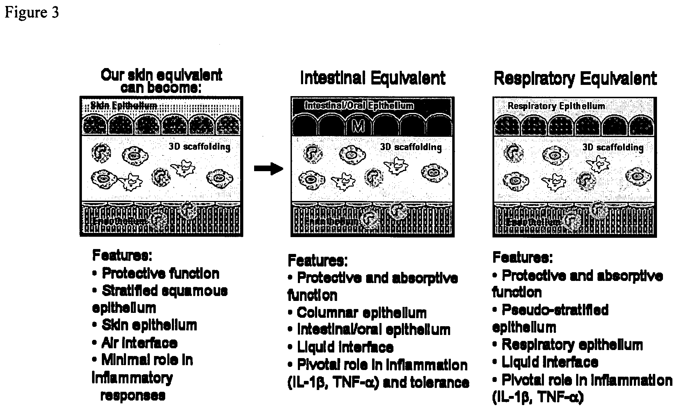

Heterogeneous tissue constructs with the addition of cells on the top and bottom of the tissue construct to form endothelial and epithelial layers. A schematic representation of the development of the generic disease model and how it can be tested with a particular disease is shown in Figure 3. As an example, we used a polycarbonate membrane support structure to prepare a 3D ECM matrix, comprising either collagen, synthetic or natural materials (*e.g*., hydrogels, PLA, PLGA, gelatin, hyaluronic acid), or combinations thereof. We have established an ECM that is capable of supporting two cell layers. We first grow a layer of epithelial cells (*e.g*., human keratinocytes) on one side of the matrix. An advantage of this model is that other epithelial cells can be used, such as respiratory epithelial cells, skin epithelial cells, or intestinal/oral epithelial cells (as schematically illustrated in Fig. 3). The basement membrane zone between the epithelium and the matrix is important to the success of this aspect of the construct and additions, such as collagen types IV or VII can be included. For a melanoma model the barrier function of the basement membrane may also be important in dissecting the pathology of modes of metastasis. This is an advantage of the general architecture of the disclosed disease model; it can be used to mimic many tissues by using different epithelial cell types. After melanocyte and keratinocyte seeding and when the keratinocytes have become established and begun stratification, the cells are exposed to an air interface, to encourage continued stratification, formation of tight cell junctions, and keratinization.

When a keratinized cell layer is formed, the construct can be inverted, so that a layer of endothelial cells *(e.g.,* HUVECs, immortalized endothelial cell lines) can be applied to the other side. When the endothelial cells have established, the construct can be inverted (so it will be upright again) to reinstate the air interface for the keratinocytes. When the endothelial cells form a confluent monolayer, the tissue construct is complete and ready for characterization.

In other embodiments of the present disclosure, in a multifunctional disease model without melanocytes in epithelial layer, a viral or bacterial disease model can be prepared. In these embodiments, either the viral or bacterial component is applied to the specialized, non-keratinized epithelial surface, mimicking normal physiologic events. In viral and bacterial invasion / infection, epithelial compromise is caused by either cellular infection or release of bacterial toxins, which can also be monitored.

### Example 4

Viability of the 3D generic disease tissue constructs. Studies of keratinocytes have shown the cells to remain viable in culture for several weeks (Boelsma et al., (2000) Acta Derm Venereol 80, 82-8). We also have experience of maintaining HUVECs in culture and on a 3D construct for several weeks. Viability of the cells on the construct can be monitored by, for example, such methods as identifying any morphological changes and by the classic LDH release assay. As cells die, the plasma membrane becomes leaky with LDH being released into the culture medium and can be measured with a coupled enzymatic assay that results in the conversion of resazurin into a fluorescent resorufin product. The amount of fluorescence produced is proportional to the number of lysed cells. Cell staining can also be performed on the tissue constructs to measure live/dead cell populations. Cell-permeant esterase substrates, such as CellTracker Green CMFDA, serve as viability probes that measure both cell membrane integrity, required for intracellular retention of the probe, and enzymatic activity, required to activate the fluorescence of the probe. Cell-impermeant nucleic acid stains, such as ethidium homodimer-1, can be used to detect dead cells. Fluorescently stained cells can then be observed by confocal microscopy.

### Example 5

Epithelial cells form stratified layers on the constructs. For the construction of the skin equivalent model, the keratinocyte layer is exposed to an air interface to encourage formation of stratified layers. The formation of the stratified layers can be monitored by microscopic examination. Periodically cell layers can be examined by using immunofluorescence confocal microscopy to identify the tight junctions and nuclei of the cells. Additionally, samples can be fixed in paraformaldehyde, embedded in parafin, cut into sections, and stained with haematoxylin and eosin for light microscopic examination.

### Example 6

Construction of a generic tissue module creating an *in vitro* disease model. In embodiments of the present disclosure, the 3D model is examined to observe immune- or inflammation-mediated responses to various diseases (*e.g*., tumors models). As examples, melanoma cells, HSV, influenza virus, *Escherichia coli,* and *Staphylococcus aureus* are used.

Melanoma cells are incorporated when the epithelial layer is formed. As human melanocytes are interfollicular, basal epithelial cells, using a cell line that is slower growing allows keratinized epithelial formation. Application of different cell types can be accomplished by intermixing these cells with normal keratinocytes (for example, at a ratio of ∼5×10³ to ∼30×10³). Monitoring of the vertical and lateral spread of the malignant melanocytes can be accomplished by staining with fluorochrome-labeled, melanocyte-specific markers and confocal microscopy. As another example, other constructs can be digested and the number of melanocytes present can be assessed using flow cytometry and similar markers.

### Example 7

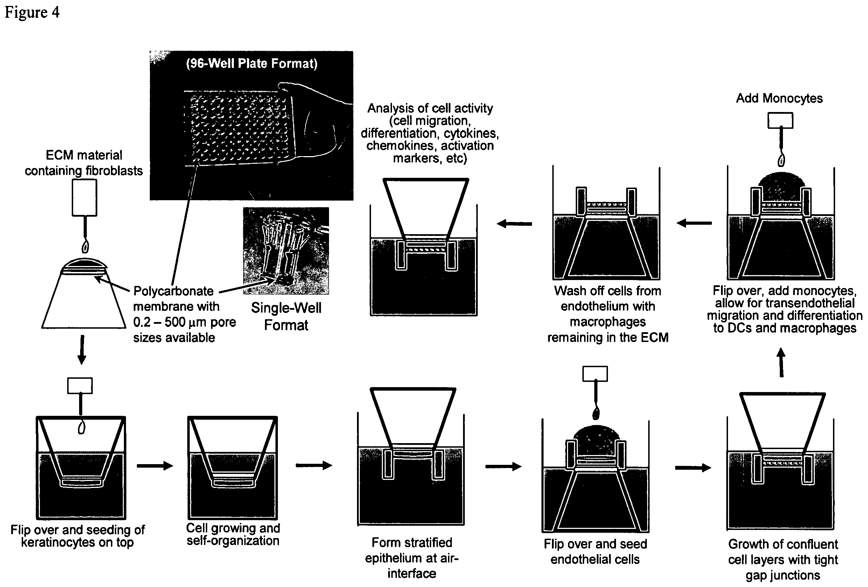

As an example, a methodology that can be used to add vertically expanding melanoma tumor cells or bacterially or virally infected fibroblast cells inside the 3D construct, is illustrated schematically in Figure 4. To add tumor cells to the disease model, we mix these cells within the ECM material before it is added to the membrane support and before we begin to grow the epithelial and endothelial cells on the matrix.

### Example 8

For the preparation of a viral model, there are several relevant methods. As an example, for a live virus, we would infect an epithelial layer. As another example, virus-infected irradiated fibroblasts can be incorporated in the collagen matrix. HLA-matched, syngeneic or autologous fibroblasts can be used; they can be propagated and infected with virus at an appropriate multiplicity of infection (MOI) (*e.g*., ∼10). Infection is allowed to proceed until an appropriate time post-infection, at which time infectious virus is UV-inactivated.

### Example 9.

*In vitro* infection/disease models are important for an analysis of the viral life cycle, including attachment, entry, and uncoating, and to unravel the interactions between viral particles and host target cells. We can also use the *in vitro* disease/infection model to examine the efficacy of the vaccine-induced immune products created in the AIS. Suitable example viral disease models include Herpes simplex viruses (HSV) and influenza viruses. Human and/or murine model systems can be used.

### Example 10

The present disclosure comprises both two- and three-dimensional (2D, 3D) models of infection/immune induction. In an example 2D model, a static culture system can be employed. In an example 3D model, the vaccination site (VS) and lymphoid tissue equivalent (LTE) can be used.

### Example 11

Several methods of viral antigen introduction are suitable for practicing the present disclosure. As an example, direct infection of cultured epithelium with virus at an appropriate multiplicity of infection (MOI) can be used. As another, example, HLA-matched or syngeneic fibroblasts can be used; they can be propagated and infected with virus at an appropriate MOI (*e.g*., ∼10). Infection will be allowed to proceed until an appropriate time post-infection at which time infectious virus will be UV-inactivated. The kinetics of virus infection and inactivation can be confirmed by, for example, immunofluorescence and plaque assay, respectively.

Infectious virus or virus-infected UV-inactivated fibroblasts can be added to the cultures. For fibroblast cultures, uninfected UV-treated fibroblasts can be used as negative controls. In 2D cultures, infectious virus, fibroblasts or vaccine/adjuvant formulations are added to a mixed immunocyte population containing antigen presenting cells (APCs) and lymphocytes. For 3D culture, antigens are introduced into a vaccination site (VS) containing reverse- transmigrated (RT) antigen presenting cells (APCs), comprising dendritic cells (DCs). APCs then process the antigen and are introduced into the lymphoid tissue equivalent (LTE), comprising T and B lymphocytes.

In both 2D and 3D cultures, immunological parameters of interest include patterns of immunocyte phenotype and cytokine synthesis and secretion. Flow cytometric analysis is valuable in this regard. Virus-specific cytotoxic activity can be assessed for T cells using, for example, a non-radioactive LDH cytoxicity assay with virus-pulsed target cells. B cells can be evaluated for specificity and isotype of antibody secretion, as well as neutralizing capability.

To evaluate recall responses and anti-viral activity, immunocytes and/or soluble factors can be recovered from 2D cultures or from the LTE of the 3D system for analysis. These immunocytes and/or biomolecules can then be tested, for example, using an *in vitro* 2D, an *in vitro* 3D tissue engineered disease model, or an *in vivo* (especially murine) disease model. In 2D experiment, these can be co-cultured with, for example, suspension or monolayer cultures of fibroblasts. The cultures can then be challenged with infectious virus or virus-infected UV-inactivated cells. As another example, a similar *in vitro* challenge can be performed in the 3D tissue engineered disease model incorporating an epithelial layer. In the *in vitro* experiments, cultures are harvested at selected times post-challenge and assayed for virus-specific immunity and anti-viral activities, as indicated, for example, by titers of infectious virus recovered.

To assess the *in vivo* efficacy of immunocytes derived from the LTE, we can conduct adoptive transfer studies in, for example, a mouse model where selected cell populations derived from the AIS can be introduced prior to viral challenge. Several murine models of HSV infection are available and can be used to assess protective efficacy of cells recovered from the AIS.

### Example 12

As another example of the present disclosure, we can conduct an 'experiment of nature' involving seropositive individuals with recurrent HSV (S⁺R⁺), seropositive individuals without recurrent disease (S⁺R⁻) and seronegative (S⁻R⁻) human subjects. Cells from these subjects can be sensitized with viral antigens. Subsequent immunological readouts can allow for discrimination of primary and recall immune events and immune profiling of protective immune mechanisms when comparing S⁺R⁺ and S⁺R⁻ subjects.

### Example 13

In a melanoma tumor model, the spread of the melanocytes radially through the epithelial layer and penetration into the sub-epithelial matrix (vertical tumor expansion) can be examined. As some melanoma cell lines exhibit radial expansion only (possibly the result of the impediment of the basement membrane structure or biochemical makeup of the different collagens) or vertical expansion only, it is possible to target the immunocyte population within the matrix. The presence of melanoma antigen with or without the addition of adjuvants, will lead to the maturation of DCs that have captured antigen.

As the APCs reverse transmigrate out of the module with captured antigens, they can be matured with TNFα. APC phenotypic markers and a panel of inflammatory cytokines can be compared to modules without melanoma cell additions. These results can then be compared to VS responses with known stimulants or adjuvants (such as LPS, CpG, poly(IC), MF59). Functional assessment of these monocyte-derived APCs after exposure to tumor antigens from the melanoma cells in the VS, can be conducted by placement into the LTE module for assessment of antigen presentation. IL12 is an important cytokine released by DCs activating T helper cells, which then release IFNγ. IFNγ contributes to CTL activity and B cell differentiation into plasma cells. Antibody release, compliment fixation, and influx of PMNs to the region of the tumor cells (*in vivo*) causes release of TNFα. TNFα and IFNγ have tumor cytostatic properties. (Croci et al. (2004) Cancer Res 64, 8428-34) and can be monitored. As an example, a non-radioactive cytotoxicity T cell assay monitoring LDH release can be used.

### Example 14.

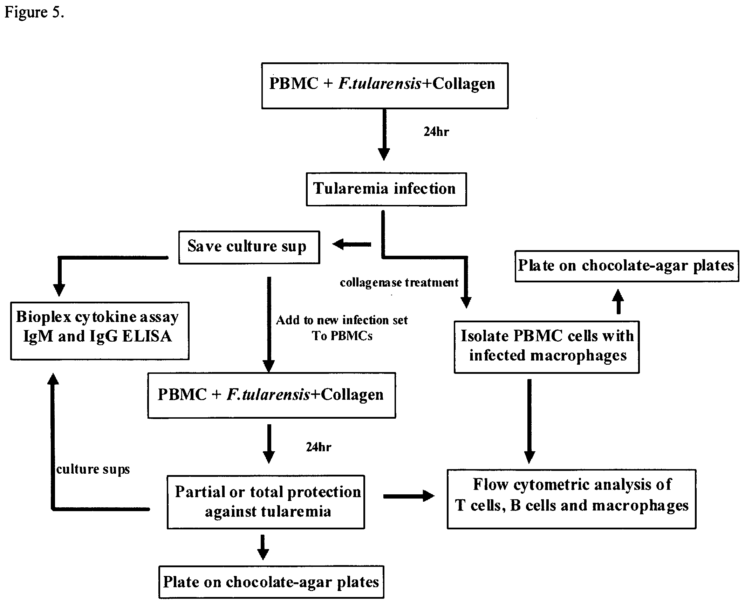

Tularemia model. In an embodiment of the disclosure, an *in vitro* model of tularemia is prepared. Live attenuated strains can be safely used in a BSL-2 laboratory. PBMCs from a series of human blood donors are prepared and mixed with different ratios of *F. tularensis* in a collagenous 3D matrix. The immune response to *F. tularensis* is then assessed, using, for example, the Bioplex 22-cytokine kit. For example, levels of IL-18, IL-1β, IFN-γ, IL-12, IL-4, and IL-5 are determined. IgM and IgG antibody ELISAs are conducted on culture supernatants to examine the humoral response. The collagenous construct is digested with collagenase to release the cells, and aliquots are serially diluted and plated on chocolate-agar plates. Colonies are counted after ∼3 days to determine the multiplication of pathogen in host cells. The activation state of T cells, B cells, and macrophages in response to infection is determined by, for example, FACS analysis. A flowsheet of the experiment is shown in Figure 5.

To determine whether protection is offered by secreted antibodies, the culture supernatants from first infection are added to a new set of PBMCs and *F. tularensis* in collagen to permit a new infection. This is followed by determining cytokine levels, ELISA, flow cytometric analysis, and colony counts, as described above. Any protection offered by antibodies is expected to be reflected in increased protective immune responses and lowering of pathogen colony counts.

This *in vitro* model can be used as test bed for vaccine and drug candidates. Quinolone drugs, such as ciprofloxacin, have been reported to be effective against tularemia (Johansson et al. (2002) Scand. J. Infect. Dis. 34, 327-30). The effect of such drugs can readily be tested by the addition of the drug to the *in vitro* model and determining any reduction in colony counts. Similarly, other drugs and vaccine candidates could be assessed for efficacy in this model system.

### Example 15.

Filovirus models. The vaccination site (VS) module of the artificial immune system of the present disclosure, comprising a confluent endothelium with monocytes and macrophages, can be used as a pathogenesis model system. For the disease module, the VS and Infection Site (IS) modules can be used interchangeably, unless otherwise noted. To prepare a filovirus disease model, as an example intracellular viral pathogen, IS modules are prepared and serve as infection targets. Preparation of the IS module comprises growing a monolayer of endothelial cells over a collagen matrix. PBMCs are added on top of the endothelial cell layer. Monocytes preferentially extravasate across the endothelium into the collagen matrix, with some T and B cells (5-10%). Monocytes in PBMCs differentiate into APCs of a range of phenotypes as they traverse the endothelium. Some monocytes differentiate into mature and immature dendritic cells (DCs) and then reverse transmigrate across the endothelium from the collagen, while other monocytes differentiate into macrophages and these sub-endothelial cells remain in the collagen matrix. The system models the *in vivo* differentiation process, whereby APCs (e.g., monocytes) entering a site of vaccination obtain differentiation signals as they cross the endothelium into the tissue. This method is superior to the widely used cytokine-induced (e.g., GM-CSF, IL-4, MCSF) methods of generating DCs and macrophages from PBMCs.

Infectious virions can be added on top of the endothelial cells. Filoviruses will infect the endothelium and also migrate across it to infect DCs and macrophages in the collagen. Additional lymphocytes can then be included in the collagen to mount an immune response against the pathogen.

Thus, the VS module/infection site (IS) module is the essential element of the Ebola disease model, the only difference is that we will incorporate additional lymphocytes. The system can be incubated for different time periods to study progression of disease. Although endothelial cells are an important part of the IS system, the presence of endothelial cells in the system may generate MHC-I-mediated allogeneic responses in response to Ebola virus or VSV expressing Ebola viral proteins or the vaccine candidates. If they do, APCs are isolated from the IS module after 48 h (when DCs and macrophages have differentiated in the IS) and add them with fresh PBMCs in the LTE module/disease module for stimulation. If the endothelial cells do not generate allogeneic responses, we will continue to use the IS as the disease module.

Thus, the VS/IS module of the artificial immune system of the present disclosure closely mimics the *in vivo* scenario of infection; all of the components involved in the infection process, including endothelial cells, DCs, macrophages and lymphocytes, are present in the model system. The system can be incubated for different time periods to study progression of disease. Cytokine profiles are assessed using, for example, a Bioplex assay and antibody responses are assessed by, for example, ELISA at different time points using culture supernatants from this model. For example, levels of IFNα□, IL-6, MCP-1, MIP-1a, MIP-1b, IFN-β, IFN-γ, IL-18, and TNF-α are determined. Disease-associated factors, such as TF, can also be measured. The collagen matrix is digested with collagenase to release the cells; their apoptotic state and expression levels of different cellular markers on endothelial cells, DCs, macrophages, and lymphocytes are assessed by, for example, flow cytometry. Antibodies generated can also be assessed.

To assess the efficacy of filovirus vaccines, PBMC cells from a series of human blood donors are incubated with different concentrations (e.g., ∼1-50 µg/mL) of, for example, Ebola vaccine in the VS module and in a simple collagenous 3D matrix and are incubated for different time periods. The vaccine formulation will be taken up by monocytes, in a similar way to infectious virions, resulting in the establishment of a host immune response to the vaccine antigens. This host immune response to the test vaccine is then assessed in terms of, for example, cytokine responses, using, for example, the Bioplex 22-cytokine kit. For example, levels of IFNa□, IL-6, MCP-1, MIP-1a, MIP-1b, IFNβ, IFNγ, IL-18, and TNFα are determined. IgM and IgG antibody ELISAs are conducted with culture supernatants to determine the levels of antibody production in response to the test vaccine. The collagenous construct is digested with collagenase to release the cells and the activation and apoptotic state of T cells, B cells and monocytes, macrophages, and dendritic cells in response to infection are determined by FACS analysis. Experiments using vectors expressing different viral proteins, peptides, and combinations of proteins can also be used to assess differences and variations in the host immune responses to viral proteins.

### Example 16.

*Yersinia* model. An embodiment of the present disclosure comprises a disease model of *Yersinia pestis* infection. In an infection module (e.g., the IS), macrophages are infected by the pathogen. The disease module models the process by which infected macrophages transport the pathogen to the regional lymph nodes. In this disease module, the lymphocytes are activated/primed and the pathogen multiplies. This lymph node equivalent (LTE) module serves as the disease module. *In vivo,* the bacteria replicate in lymph nodes before migrating to other organs. The disclosed artificial immune system is flexible in that the various modules are designed as plug-and-play immunological constructs. This feature is exploited here with infection and disease modules of the *Yersinia* infectious disease model.

The infection module system (IS) involves growing a monolayer of endothelial cells over a collagen matrix. PBMCs are added on top of the endothelial cell layer. Monocytes preferentially extravasate across the endothelium into the collagen matix with a small number of T and B cell lymphocytes (5-10%). The monocytes in the PBMCs differentiate into APCs of a range of phenotypes as they traverse the endothelium. Some monocytes differentiate into mature and immature dendritic cells (DCs) and reverse transmigrate across the endothelium from the collagen, while other monocytes differentiate into macrophages and these sub-endothelial cells remain in the collagen matrix. This system mimics the *in vivo* differentiation process, where antigen-presenting cells (e.g., monocytes) entering the site of vaccination receive differentiation signals as they cross the endothelium into the tissue.

To prepare the disease model, *Y. pestis* is added at different multiplicities of infection (MOI) on top of the endothelial cells of the IS module. *Y. pestis* crosses the endothelium and enters the collagen where the pathogen will infect the macrophages and also pulse the dendritic cells with *Y. pestis* antigens. After 4-6 h of infection, the collageneous matrix is digested to release the APCs.

These infected APCs from the IS are then transferred to the disease module (LTE) where they are added with autologous PBMCs and can be co-cast in collagen. The lymphocytes in collagen mount an immune response against the pathogen. The system can be incubated for different time periods to study disease progression. The disease manifestation is observed in terms of, for example, the development of severe necrotic inflammation, macrophages and dendritic cells undergoing apoptosis as a result of infection and inflammation, and the host immune system mounting an immune response to contain the infection. Containment of infection will be reflected as reduction in bacterial counts. Host macrophages, DCs, and lymphocytes will secrete pro-inflammatory cytokines and chemokines and antibodies in response to the infection.

Parameters of disease pathogenesis will be determined as follows. Collagen constructs from the disease module are paraffin-embedded followed by sectioning and microscopy to examine the inflammatory lesions. Collagen is digested with collagenase to release cells, which are then assayed for apoptotic and other cellular expression markers by flow cytometry. This will provide information about host cell death and the activation profile of the immune cells due to the infection. An aliquot of the collagenase digest will be plated on nutrient agar plates to determine the colony forming units (cfu) and hence survival and multiplication of pathogen in the host cells and containment of the infection. Culture supernatants are assayed for cytokine and antibody secretion. The cytokine profiles are determined by, for example, a Bioplex assay and antibody responses are assessed, for example, by ELISA at different time points. For example, levels of IL-6, MCP-1, IL-12p35, IFNγ and TNFα can be determined. IgM and IgG antibody ELISAs are conducted using culture supernatants to determine the levels of antibody production in response to the infection.

In further embodiment of the present disclosure, the infectious disease model is used to test potential therapeutic methods that may cure the infection, pre- and/or post-exposure. The model can also be used to address basic questions related to bacterial pathogenesis.

The effects of various TLR agonists and vaccine adjuvants have been examined using the VS system. It has been reported that human monocytes infected with *Y. pestis* express cell surface TLR9 and differentiate into dendritic cells (Shaikh 2004). The effects of CpG and/or other adjuvants and *Y. pestis* infection in modulating DC function as APCs and on ability to contain the infection, can readily be tested in the model system of the present disclosure by pretreating the infection module with TLR agonists for 24-48 h, followed by introduction of the pathogen for 4-6 h. Infected APCs are taken out of the infection module and are then added to the disease module for different periods. The collagenous matrix from the disease module is digested with collagenase. The protection offered by, for example, adjuvants can be quantitated by plating the collagenase digest (as described above) and estimating the reduction in cfu.

Cytokines play important roles in the control of infection. Extraneous IFNγ and/or TNFα or IL-12 can be added in different concentrations to the disease module together with APCs from the infection module. The control of infection can be studied, for example, in terms of reduction in cfu by, for example, plating method and by, for example, determining cytokine and antibody levels in culture supernatants.

The TTSS and Yops are important for *Y. pestis* pathogenesis, as discussed earlier. Their effects can be tested in the infection module by adding these antigens to it and transferring the affected APCs to the disease module and determining the ability to contain the infection, by, for example, measuring the reduction in cfu by, for example, a plating method.

Monoclonal antibodies have been produced against *Y. pestis* and have been reported to protect Balb/c mice against *Y. pestis* challenge (Eyles et al. (2007) Vaccine 25, 7301-6). The protective effect of these monoclonals can be tested in the disease module system by adding protective or therapeutic antibodies to it. The infected APCs from the infection module, together with protective antibodies and autologous PBMCs, can then be co-cast in the disease module for different periods. Protection by the antibodies is determined, for example, by reduction in cfu by, for example, a plating method. Additionally, antibodies in the culture supernatants in the disease module, produced in response to the infection, can be added to a new infection set and any protection offered can be determined, for example, by cfu reduction, using, for example, a plating method.

In another embodiment of the present disclosure, vaccine candidates, such as those using the F1 or V antigen, can be tested for efficacy by placing *in vitro*-immunized lymphocytes in the disease module. First, the vaccine is placed in the VS module to prime APCs. These APCs are then placed in the LTE module to prime lymphocytes, which will be used in the disease module. These *in vitro*-immunized lymphocytes are placed in the disease module along with infected APCs (obtained from a parallel infection with *Y. pestis* in another infection module). Autologous PBMCs will be used throughout. The protection offered can then be quantitated, by for example, reduction in bacterial cfu by, for example, a plating method.

### Example 17.

Burkholderia model. An embodiment of the present disclosure comprises an *in vitro* disease model of *Burkholderia mallei* using the artificial immune system, based on multidimensional interrogation of human leukocytes. The artificial immune system can rapidly provide information about the effects of an immunotherapy on human population subgroups (genetic diversity, HLA haplotypes, age, gender).

In the infection module (IS) of the artficial immune system of the present disclosure, macrophages are infected by the pathogen (the IS module). The disease module models the process whereby infected macrophages transport the pathogen to the regional lymph nodes. In this disease module, lymphocytes are activated/primed and the pathogen multiplies. This LTE module is the disease module of the *in vitro* system. The bacteria replicate in lymph nodes before migrating to other organs. The artificial immune system is flexible in that the various modules are 'plug-and-play; immunological constructs. This feature is exploited in an embodiment of the present disclosure, comprising the infection and disease modules of a *Burkholderia* infectious disease model.

Preparation of the infection site module involves growing a monolayer of endothelial cells over a collagen matrix. PBMCs are added on top of the endothelial cell layer. Monocytes preferentially extravasate across the endothelium into the collagen matrix with a small number of T and B cells (∼5-10%). The monocytes in the PBMCs differentiate into APCs of a range of phenotypes as they traverse the endothelium. Some monocytes differentiate into mature and immature dendritic cells (DCs) and reverse transmigrate across the endothelium from the collagen, while other monocytes differentiate into macrophages and these sub-endothelial cells remain in the collagen matrix. The system mimics the *in vivo* differentiation process whereby antigen-presenting cells (e.g., monocytes) entering the site of vaccination receive differentiation signals as they cross the endothelium into the tissue.

In the IS, cells are infected by adding *B. mallei* at different multiplicities of infection (MOI) on top of the endothelial cells. *B. mallei* crosses the endothelium and enters the collagen where the pathogen will infect the macrophages and also pulse the dendritic cells with *B. mallei* antigens. After 4-6 h of infection, the collageneous matrix will be digested to release the APCs.

These infected APCs from the IS will then be transferred to the disease module where they are added with autologous PBMCs and can be co-cast in collagen. The lymphocytes in collagen mount an immune response against the pathogen. The system can be incubated for different time periods to study disease progression. The disease manifestation is observed in terms of measurable events or parameters, such as development of necrotizing lesions, macrophage and lymphocyte death, by apoptosis, as a result of infection and inflammation, and the host immune response to contain the infection. Containment of infection will be reflected as a reduction in bacterial counts. Host macrophages, DCs, and lymphocytes will secrete proinflammatory cytokines and chemokines and also antibodies in response to the infection; these can also be assessed.

Parameters of disease pathogenesis will be determined. The collagen constructs from the disease module will be paraffin-embedded, followed by sectioning and microscopy to visualize necrotic lesions. Collagen will be digested with collagenase to release cells, which will be then assayed for apoptotic and other cellular expression markers by flow cytometry. This will provide information on host cell death and the activation profile of the immune cells due to the infection. An aliquot of the collagenase digest will be plated on Mueller-Hinton agar plates to determine the colony forming units (cfu) and hence survival and multiplication of the pathogen in the host cells, and the containment of the infection. Culture supernatants will be assayed for cytokine and antibody secretion. The cytokine profiles can be determined, for example, by a Bioplex assay and antibody responses can be examined, for example, by ELISA at different time points. For example, levels of IL-6, MCP-1, IL-12p35, IFNγ, IL-18, IP-10, and TNFα can be determined. IgM and IgG antibody ELISA can be conducted using culture supernatants to determine the levels of antibody production in response to the infection.

In another embodiment of the present disclosure, the infectious disease model can be used to test potential therapies that may cure the infection pre- and/or post-exposure. The model may also be used to address basic questions related to bacterial pathogenesis.

Studies with different types of TLR agonists and vaccine adjuvants have been conducted using the VS system. The protective effects of CpG and/or other adjuvants can readily be tested by pretreating the infection module with TLR agonists for 24-48 h, followed by introduction of the pathogen for 4-6 h. Infected APCs are taken out of the infection module and are then added the disease module for different periods. The collagenous matrix from disease module can be digested with collagenase. The protection offered by the adjuvants will be quantitated by plating the collagenase digest (as described above) and estimating the reduction in cfu.

As type I cytokines are important in control of initial infection (described above), extraneous IFNγ and/or IL-12 will be added in different concentrations to the disease module together with APCs from infection module. The control of infection will be studied in terms of reduction in cfu, by, for example, a plating method, and also the cytokine and antibody levels determined in culture supernatants.

The animal-type TTSS is important for *B. mallei* pathogenesis. This can be tested in the infection and disease module by using RD01 and RD02 mutant strains (Ribot & Ulrich (2006) Infect. Immun. 74, 4349-53).

Monoclonal antibodies have been produced againt *B. mallei* and reported to passively protect Balb/c mice against *B. mallei* aerosol challenge (Treviño et al. (2006) Infect. Immun. 74, 1958-61). The protective effect of these monoclonals can be tested in the disease module system by adding protective or therapeutic antibodies to it. Infected APCs from the infection module together with protective antibodies and autologous PBMCs can be co-cast in the disease module for different periods. Protection by the antibodies is determined by reduction in cfu, by, for example, a plating method. Additionally, the antibodies in the culture supernatants in the disease module, produced in response to the infection, can be added to a new infection set and any protection offered can be determined in terms of reduction in cfu, by, for example, a plating method.

Vaccine candidates can be tested in the disease model by placing *in vitro*-immunized lymphocytes in the disease module. First, the vaccine is placed in the VS module to prime APCs. These APCs are then placed in the LTE module to prime lymphocytes, which are then used in the disease module. These *in vitro*-immunized lymphocytes are placed in the disease module along with infected APCs (obtained from a parallel infection with *B. mallei* in the IS module). Autologous PBMCs are used throughout. The protection offered is quantitated by reduction in bacterial cfu, by, for example, a plating method.

In another embodiment of the present disclosure, the disease model can be used to test new drug, vaccine, or therapeutic candidates for their efficacy against glanders or melioidosis, diseases caused by *Burkholderia mallei* and *B. pseudomallei.*

### Example 18.

HIV models. HIV (human immunodeficiency virus) is the virus that causes AIDS (acquired immune deficiency syndrome). *In vivo,* HIV-1 infects DCs and they move to the lymph nodes where the virus infects CD4⁺ T cells. The lymph node then becomes the principal site of virus production. In embodiments of the present disclosure, the steps of this process are modeled by the modules of the artificial immune system.

In an embodiment of the present disclosure, HIV vaccine candidates are tested. As an example, HIV gp120 or gp140 vaccines can be assessed. HIV gp120/gp140-specific responses are assessed using PBMCs from human blood donors. First, the candidate vaccine is placed in the VS module to prime APCs. These primed APCs are then placed in the LTE module to prime lymphocytes. The *in vitro*-immunized lymphocytes and antibodies generated in the LTE are placed in the disease module along with infected APCs (obtained from a parallel infection with HIV in the infection site module). Autologous PBMCs are used throughout. Any protection offered is quantitated by, for example, assessing inhibition of viral replication or cell lysis.

Preparation of the infection site (IS) module involves growing a monolayer of endothelial cells over a collagen matrix. PBMCs are added on top of the endothelial cell layer. Monocytes preferentially extravasate across the endothelium into the collagen matix with a small number of T and B cells (∼5-10%). The monocytes in the PBMCs differentiate into APCs of a range of phenotypes as they traverse the endothelium. Some monocytes differentiate into mature and immature dendritic cells (DCs) and reverse transmigrate across the endothelium from the collagen, while other monocytes differentiate into macrophages and these sub-endothelial cells remain in the collagen matrix. The system mimics the *in vivo* differentiation process whereby antigen-presenting cells (e.g., monocytes) entering the site of infection receive differentiation signals as they cross the endothelium into the tissue. This method is superior to the widely used cytokine-induced (e.g., GM-CSF, IL-4, MCSF) methods of generating DCs and macrophages from PBMCs.

In an embodiment of the present disclosure, infectious virions can be added on top of the endothelial cells. HIV will infect the antigen-presenting cells. Thus, the IS module of the artificial immune system of the present disclosure closely mimics the *in vivo* scenario of infection; all of the components involved in the infection process, including endothelial cells, DCs, macrophages and lymphocytes, are present in the model system. Infected APCs can then be transferred to the LTE module.

The system can be incubated for different time periods to study progression of disease. Cytokine profiles can be assessed using a Bioplex assay and antibody responses can be assessed by ELISA at different time points using culture supernatants. For example, levels of IFNα, IFN-γ, IL-18, IL-6, MCP-1, MIP-1a, MIP-1b, IFN-β, and TNF-α will be determined.

To assess the efficacy of HIV vaccines, PBMC cells from a series of human blood donors are incubated with different concentrations (e.g., ∼1-50 µg/mL) of HIV vaccine in the VS module and in a simple collagenous 3D matrix and are incubated for different time periods. The vaccine formulation will be taken up by monocytes, in a similar way to infectious virions, resulting in the establishment of a host immune response to the vaccine antigens. This host immune response to the test vaccine is then assessed in terms of, for example, cytokine responses, using for example, the Bioplex 22-cytokine kit. For examples, levels of IL-6, MCP-1, MIP-1a, MIP-1b, IFNβ, IFNγ, IL-18, and TNFα are determined. IgM and IgG antibody ELISAs are conducted with culture supernatants to determine the levels of antibody production in response to the test vaccine. If the LTE comprises a collagen matrix, the collagenous construct is digested with collagenase to release the cells and the activation and apoptotic state of T cells, B cells, and monocytes, macrophages, and dendritic cells in response to infection are determined by FACS analysis. Experiments using vectors expressing different viral proteins, peptides, and combinations of proteins can also be used to assess differences and variations in the host immune responses to viral proteins.

In the IS, PBMCs are infected by adding HIV at different multiplicities of infection (MOI) on top of the endothelial cells. HIV infects antigen-presenting cells in the IS. These infected APCs are then transferred to the disease module/LTE module described above where they are added with autologous PBMCs and can be co-cast in collagen. The lymphocytes mount an immune response against the pathogen. The system can be incubated for different time periods to study disease progression. The disease manifestation is observed in terms of measurable events or parameters, such as development of macrophages and lymphocytes death and the host immune response to contain the infection. Containment of infection will be reflected as a reduction in viral counts. Host macrophages, DCs, and lymphocytes will secrete proinflammatory cytokines and chemokines and also antibodies in response to the infection; these can also be assayed in culture supernatants.

The cytokine profiles can be determined, for example, by a Bioplex assay and antibody responses can be examined, for example, by ELISA at different time points. For example, levels of IL-6, MCP-1, IL-12p35, IFNγ, IL-18, IP-10, and TNFα can be determined. IgM and IgG antibody ELISA can be conducted using culture supernatants to determine the levels of antibody production in response to the infection.

In an embodiment of the present disclosure, diseased cells (e.g., HIV-infected APCs) would be exposed to 'output' from the VS/LTE combination - either by transferring VS/LTE 'output' (Fig. 11) or by putting the diseased cells (e.g., HIV-infected APCs) into the LTE (Fig. 12), in both cases, to evaluate the effect on the diseased cells. The effects on the LTE components by the diseased cells can be assessed, as can any effect on the diseased cells.

In an embodiment of the disclosure, IS-derived cells would be infected before putting them into a naive (uninfected) LTE, to assess the effects on the cells of the LTE. This models cells becoming infected in the periphery before moving to the lymph node (or LTE). Effects on the LTE components by the diseased cells can be assessed, as can effects on the diseased cells.

In another embodiment of the present disclosure, a naive (uninfected) LTE is infected to assess the effects on cells of the LTE.

## Claims

1. An artificial immune system to permit the assessment of agents *in vitro* without administration to animal subjects, comprising a cell culture comprising:
a two- or three-dimensional matrix, comprising lymphoid tissue; and
diseased cells, wherein said diseased cells are immune system cells, selected from the group consisting of PBMCs, monocytes, macrophages, dendritic cells, lymphocytes, and antigen-presenting cells, and wherein said immune system cells are virally-infected cells, bacterially-infected cells, tumor cells, or autoimmune disease-afflicted cells.

2. The artificial immune system of claim 1, wherein the cell culture further comprises:
a three-dimensional matrix, comprising epithelial and/or endothelial cells.

3. The artificial immune system of claim 1 or claim 2, wherein said diseased cells are virally infected cells or bacterially infected cells.

4. The artificial immune system of claim 1 or claim 2, wherein said diseased cells comprise PBMCs derived from blood donors infected with a virus or a bacterium.

5. The artificial immune system of claim 1 or claim 2, wherein said diseased cells comprise PBMCs derived from uninfected blood donors that are then infected in vitro with a virus or a bacterium.

6. The artificial immune system of claim 1 or claim 2, wherein said diseased cells comprise PBMCs derived from blood donors afflicted with an autoimmune disease.

7. The artificial immune system of any one of claims 1 to 6, wherein said agent is selected from the group consisting of vaccines, adjuvants, immunotherapy candidates, cosmetics, drugs, biologics, and chemical compounds.

8. The artificial immune system of claim 7, wherein said agent is a vaccine.

9. A method of evaluating *in vitro* the potential reaction of an animal to an agent, said method comprising:
administering an agent to the artificial immune system of claim 1 or claim 2, and
evaluating the effects on the diseased cells, the lymphoid tissue or the cells of the lymphoid tissue.

10. A method of evaluating *in vitro* the potential reaction of an animal to an agent, said method comprising:
administering an agent to a three-dimensional matrix, comprising epithelial and/or endothelial cells;
co-culturing said three-dimensional matrix comprising epithelial and/or endothelial cells with a three-dimensional matrix comprising lymphoid tissue;
adding diseased cells to the co-culture, wherein said diseased cells are immune system cells, selected from the group consisting of PBMCs, monocytes, macrophages, dendritic cells, lymphocytes, and antigen-presenting cells, and wherein said immune system cells are virally-infected cells, bacterially-infected cells, tumor cells, or autoimmune disease-afflicted cells; and
evaluating the effects of the agent on the diseased cells.

11. A method of evaluating *in vitro* the potential reaction of an animal to an agent, said method comprising:
administering an agent to a three-dimensional matrix, comprising epithelial and/or endothelial cells;
co-culturing said three-dimensional matrix comprising epithelial and/or endothelial cells with a two- or three-dimensional matrix comprising lymphoid tissue;
adding diseased cells, wherein said diseased cells are immune system cells, selected from the group consisting of PBMCs, monocytes, macrophages, dendritic cells, lymphocytes, and antigen-presenting cells, and wherein said immune system cells are virally-infected cells, bacterially-infected cells, tumor cells, or autoimmune disease-afflicted cells, to the co-culture; and
evaluating the effects on cells of the co-culture.

12. A method of evaluating *in vitro* the potential reaction of an animal to an agent, said method comprising:
administering an agent to the artificial immune system of Claim 2, and
co-culturing the three-dimensional matrix comprising epithelial and/or endothelial cells with a three-dimensional matrix comprising lymphoid tissue;
adding culture media obtained from the co-culture to diseased cells, wherein said diseased cells are immune system cells, selected from the group consisting of PBMCs, monocytes, macrophages, dendritic cells, lymphocytes, and antigen-presenting cells, and wherein said immune system cells are virally-infected cells, bacterially-infected cells, tumor cells, or autoimmune disease-afflicted cells; and
evaluating the effects on the diseased cells.

13. A method of evaluating *in vitro* the potential reaction of an animal to an agent, said method comprising:
administering an agent to a two- or three-dimensional matrix comprising epithelial and/or endothelial cells;
co-culturing the three-dimensional matrix comprising epithelial and/or endothelial cells with a two- or three-dimensional matrix comprising lymphoid tissue;
adding culture media obtained from the co-culture to diseased cells wherein said diseased cells are immune system cells, selected from the group consisting of PBMCs, monocytes, macrophages, dendritic cells, lymphocytes, and antigen-presenting cells, and wherein said immune system cells are virally-infected cells, bacterially-infected cells, tumor cells, or autoimmune disease-afflicted cells; and
evaluating the effects on the diseased cells.

14. The method of any one of claims 10 to 13, wherein the animal is a diseased animal.

15. The method of any one of claims 10 to 14, wherein said agent is selected from the group consisting of vaccines, adjuvants, immunotherapy candidates, cosmetics, drugs, biologics, and chemical compounds.

16. The method of claim 15, wherein said agent is a vaccine.

## Patentansprüche

1. Ein künstliches Immunsystem zum Erlauben der Bewertung von Wirkstoffen in vitro ohne Verabreichung an Tiere, umfassend eine Zellkultur, die umfasst:
eine zwei- oder dreidimensionale Matrix, die lymphatisches Gewebe umfasst; und
kranke Zellen, wobei die besagten kranken Zellen Immunsystemzellen sind, die ausgewählt sind aus der Gruppe bestehend aus PBMCs, Monozyten, Makrophagen, dendritischen Zellen, Lymphozyten und antigenpräsentierenden Zellen und wobei die besagten Immunsystemzellen viral infizierte Zellen, bakteriell infizierte Zellen, Tumorzellen oder von einer Autoimmunerkrankung befallene Zellen sind.

2. Das künstliche Immunsystem nach Anspruch 1, wobei die Zellkultur weiter umfasst:
eine dreidimensionale Matrix, die Epithel- und/oder Endothelzellen umfasst.

3. Das künstliche Immunsystem nach Anspruch 1 oder Anspruch 2, wobei die besagten kranken Zellen viral infizierte Zellen oder bakteriell infizierte Zellen sind.

4. Das künstliche Immunsystem nach Anspruch 1 oder Anspruch 2, wobei die besagten kranken Zellen PBMCs umfassen, die von mit einem Virus oder einem Bakterium infizierten Blutspendern abgeleitet sind.

5. Das künstliche Immunsystem nach Anspruch 1 oder Anspruch 2, wobei die besagten kranken Zellen PBMCs umfassen, die von nicht infizierten Blutspendern abgeleitet sind und die dann in vitro mit einem Virus oder einem Bakterium infiziert werden.

6. Das künstliche Immunsystem nach Anspruch 1 oder Anspruch 2, wobei die besagten kranken Zellen PBMCs umfassen, die von Blutspendern abgeleitet sind, die an einer Autoimmunerkrankung leiden.

7. Das künstliche Immunsystem nach einem der Ansprüche 1 bis 6, wobei der besagte Wirkstoff ausgewählt ist aus der Gruppe bestehend aus Impfstoffen, Adjuvanzien, Immuntherapiekandidaten, Kosmetika, Medikamenten, Biologika und chemischen Verbindungen.

8. Das künstliche Immunsystem nach Anspruch 7, wobei der besagte Wirkstoff ein Impfstoff ist.

9. Ein Verfahren zur In-Vitro-Untersuchung der möglichen Reaktion von einem Tier auf einen Wirkstoff, wobei das besagte Verfahren umfasst:
Verabreichen eines Wirkstoffs an das künstliche Immunsystem nach Anspruch 1 oder Anspruch 2, und
Untersuchen der Wirkungen auf die kranken Zellen, das lymphatische Gewebe oder die Zellen von dem lymphatischen Gewebe.

10. Ein Verfahren zur In-Vitro-Untersuchung der möglichen Reaktion von einem Tier auf einen Wirkstoff, wobei das besagte Verfahren umfasst:
Verabreichen eines Wirkstoffs an eine dreidimensionale Matrix, die Epithel- und/oder Endothelzellen umfasst;
Co-Kultivieren der besagten dreidimensionalen Matrix, die Epithel- und/oder Endothelzellen umfasst, mit einer dreidimensionalen Matrix, die lymphatisches Gewebe umfasst;
Hinzugeben von kranken Zellen zu der Co-Kultur, wobei die besagten kranken Zellen Immunsystemzellen sind, die ausgewählt sind aus der Gruppe bestehend aus PBMCs, Monozyten, Makrophagen, dendritischen Zellen, Lymphozyten und antigenpräsentierenden Zellen und wobei die besagten Immunsystemzellen viral infizierte Zellen, bakteriell infizierte Zellen, Tumorzellen oder von einer Autoimmunerkrankung befallene Zellen sind; und
Untersuchen der Wirkungen von dem Wirkstoff auf die kranken Zellen.

11. Ein Verfahren zur In-Vitro-Untersuchung der möglichen Reaktion von einem Tier auf einen Wirkstoff, wobei das besagte Verfahren umfasst:
Verabreichen eines Wirkstoffs an eine dreidimensionale Matrix, die Epithel- und/oder Endothelzellen umfasst;
Co-Kultivieren der besagten dreidimensionalen Matrix, die Epithel- und/oder Endothelzellen umfasst, mit einer zwei- oder dreidimensionalen Matrix, die lymphatisches Gewebe umfasst;
Hinzugeben von kranken Zellen, wobei die besagten kranken Zellen Immunsystemzellen sind, die ausgewählt sind aus der Gruppe bestehend aus PBMCs, Monozyten, Makrophagen, dendritischen Zellen, Lymphozyten und antigenpräsentierenden Zellen und wobei die besagten Immunsystemzellen viral infizierte Zellen, bakteriell infizierte Zellen, Tumorzellen oder von einer Autoimmunerkrankung befallene Zellen sind; und
Untersuchen der Wirkungen auf Zellen von der Co-Kultur.

12. Ein Verfahren zur In-Vitro-Untersuchung der möglichen Reaktion von einem Tier auf einen Wirkstoff, wobei das besagte Verfahren umfasst:
Verabreichen eines Wirkstoffs an ein künstliches Immunsystem nach Anspruch 2, und
Co-Kultivieren der dreidimensionalen Matrix, die Epithel-und/oder Endothelzellen umfasst, mit einer dreidimensionalen Matrix, die lymphatisches Gewebe umfasst;
Hinzugeben von von der Co-Kultur erhaltenen Kulturmedien zu kranken Zellen, wobei die besagten kranken Zellen Immunsystemzellen sind, die ausgewählt sind aus der Gruppe bestehend aus PBMCs, Monozyten, Makrophagen, dendritischen Zellen, Lymphozyten und antigenpräsentierenden Zellen und wobei die besagten Immunsystemzellen viral infizierte Zellen, bakteriell infizierte Zellen, Tumorzellen oder von einer Autoimmunerkrankung befallene Zellen sind; und
Untersuchen der Wirkungen auf die kranken Zellen.

13. Ein Verfahren zur In-Vitro-Untersuchung der möglichen Reaktion von einem Tier auf einen Wirkstoff, wobei das besagte Verfahren umfasst:
Verabreichen eines Wirkstoffs an eine zwei- oder dreidimensionale Matrix, die Epithel- und/oder Endothelzellen umfasst;
Co-Kultivieren der dreidimensionalen Matrix, die Epithel-und/oder Endothelzellen umfasst, mit einer zwei- oder dreidimensionalen Matrix, die lymphatisches Gewebe umfasst;
Hinzugeben von von der Co-Kultur erhaltenen Kulturmedien zu kranken Zellen, wobei die besagten kranken Zellen Immunsystemzellen sind, die ausgewählt sind aus der Gruppe bestehend aus PBMCs, Monozyten, Makrophagen, dendritischen Zellen, Lymphozyten und antigenpräsentierenden Zellen und wobei die besagten Immunsystemzellen viral infizierte Zellen, bakteriell infizierte Zellen, Tumorzellen oder von einer Autoimmunerkrankung befallene Zellen sind; und
Untersuchen der Wirkungen auf die kranken Zellen.

14. Das Verfahren nach einem der Ansprüche 10 bis 13, wobei das Tier ein krankes Tier ist.

15. Das Verfahren nach einem der Ansprüche 10 bis 14, wobei der besagte Wirkstoff ausgewählt ist aus der Gruppe bestehend aus Impfstoffen, Adjuvanzien, Immuntherapiekandidaten, Kosmetika, Medikamenten, Biologika und chemischen Verbindungen.

16. Das Verfahren nach Anspruch 15, wobei der besagte Wirkstoff ein Impfstoff ist.

## Revendications

1. Système immunitaire artificiel destiné à permettre l'évaluation d'agents *in vitro* sans administration à des sujets animaux, comprenant une culture cellulaire comprenant :
une matrice bi- ou tri-dimensionnelle, comprenant un tissu lymphoïde ; et
des cellules malades, dans lequel lesdites cellules malades sont des cellules du système immunitaire, choisies dans le groupe constitué de cellules mononucléaires de sang périphérique, monocytes, macrophages, cellules dendritiques, lymphocytes et cellules présentant un antigène, et dans lequel lesdites cellules du système immunitaire sont des cellules infectées par virus, des cellules infectées par bactéries, des cellules tumorales, ou des cellules atteintes d'une maladie auto-immune.

2. Système immunitaire artificiel selon la revendication 1, dans lequel la culture cellulaire comprend en outre :
une matrice tridimensionnelle, comprenant des cellules épithéliales et/ou endothéliales.

3. Système immunitaire artificiel selon la revendication 1 ou la revendication 2, dans lequel lesdites cellules malades sont des cellules infectées par virus ou des cellules infectées par bactéries.

4. Système immunitaire artificiel selon la revendication 1 ou la revendication 2, dans lequel lesdites cellules malades comprennent des cellules mononucléaires de sang périphérique dérivées de donneurs de sang infectés avec un virus ou une bactérie.

5. Système immunitaire artificiel selon la revendication 1 ou la revendication 2, dans lequel lesdites cellules malades comprennent des cellules mononucléaires de sang périphérique dérivées de donneurs de sang non infectés qui sont ensuite infectés *in vitro* avec un virus ou une bactérie.

6. Système immunitaire artificiel selon la revendication 1 ou la revendication 2, dans lequel lesdites cellules malades comprennent des cellules mononucléaires de sang périphérique dérivées de donneurs de sang atteints d'une maladie auto-immune.

7. Système immunitaire artificiel selon l'une quelconque des revendications 1 à 6, dans lequel ledit agent est choisi dans le groupe constitué de vaccins, adjuvants, candidats immunothérapeutiques, cosmétiques, médicaments, agents biologiques et composés chimiques.

8. Système immunitaire artificiel selon la revendication 7, dans lequel ledit agent est un vaccin.

9. Procédé d'évaluation *in vitro* de la réaction potentielle d'un animal à un agent, ledit procédé comprenant :
l'administration d'un agent au système immunitaire artificiel selon la revendication 1 ou la revendication 2, et
l'évaluation des effets sur les cellules malades, le tissu lymphoïde ou les cellules du tissu lymphoïde.

10. Procédé d'évaluation *in vitro* de la réaction potentielle d'un animal à un agent, ledit procédé comprenant :
l'administration d'un agent à une matrice tridimensionnelle, comprenant des cellules épithéliales et/ou endothéliales ;
la co-culture de ladite matrice tridimensionnelle comprenant des cellules épithéliales et/ou endothéliales avec une matrice tridimensionnelle comprenant un tissu lymphoïde ;
l'ajout de cellules malades à la co-culture, dans lequel lesdites cellules malades sont des cellules du système immunitaire, choisies dans le groupe constitué de cellules mononucléaires de sang périphérique, monocytes, macrophages, cellules dendritiques, lymphocytes et cellules présentant un antigène, et dans lequel lesdites cellules du système immunitaire sont des cellules infectées par virus, des cellules infectées par bactéries, des cellules tumorales, ou des cellules atteintes d'une maladie auto-immune ; et
l'évaluation des effets de l'agent sur les cellules malades.

11. Procédé d'évaluation *in vitro* de la réaction potentielle d'un animal à un agent, ledit procédé comprenant :
l'administration d'un agent à une matrice tridimensionnelle, comprenant des cellules épithéliales et/ou endothéliales ;
la co-culture de ladite matrice tridimensionnelle comprenant des cellules épithéliales et/ou endothéliales avec une matrice bi- ou tridimensionnelle comprenant un tissu lymphoïde ;
l'ajout de cellules malades, dans lequel lesdites cellules malades sont des cellules du système immunitaire, choisies dans le groupe constitué de cellules mononucléaires de sang périphérique, monocytes, macrophages, cellules dendritiques, lymphocytes et cellules présentant un antigène, et dans lequel lesdites cellules du système immunitaire sont des cellules infectées par virus, des cellules infectées par bactéries, des cellules tumorales, ou des cellules atteintes d'une maladie auto-immune, à la co-culture ; et
l'évaluation des effets sur les cellules de la co-culture.

12. Procédé d'évaluation *in vitro* de la réaction potentielle d'un animal à un agent, ledit procédé comprenant :
l'administration d'un agent au système immunitaire artificiel selon la revendication 2, et
la co-culture de la matrice tridimensionnelle comprenant des cellules épithéliales et/ou endothéliales avec une matrice tridimensionnelle comprenant un tissu lymphoïde ;
l'ajout du milieu de culture obtenu à partir de la co-culture aux cellules malades, dans lequel lesdites cellules malades sont des cellules du système immunitaire, choisies dans le groupe constitué de cellules mononucléaires de sang périphérique, monocytes, macrophages, cellules dendritiques, lymphocytes et cellules présentant un antigène, et dans lequel lesdites cellules du système immunitaire sont des cellules infectées par virus, des cellules infectées par bactéries, des cellules tumorales, ou des cellules atteintes d'une maladie auto-immune, à la co-culture ; et
l'évaluation des effets sur les cellules malades.

13. Procédé d'évaluation *in vitro* de la réaction potentielle d'un animal à un agent, ledit procédé comprenant :
l'administration d'un agent à une matrice bi- ou tridimensionnelle, comprenant des cellules épithéliales et/ou endothéliales ;
la co-culture de la matrice tridimensionnelle comprenant des cellules épithéliales et/ou endothéliales avec une matrice bi- ou tridimensionnelle comprenant un tissu lymphoïde ;
l'ajout du milieu de culture obtenu à partir de la co-culture aux cellules malades, dans lequel lesdites cellules malades sont des cellules du système immunitaire, choisies dans le groupe constitué de cellules mononucléaires de sang périphérique, monocytes, macrophages, cellules dendritiques, lymphocytes et cellules présentant un antigène, et dans lequel lesdites cellules du système immunitaire sont des cellules infectées par virus, des cellules infectées par bactéries, des cellules tumorales, ou des cellules atteintes d'une maladie auto-immune, à la co-culture ; et
l'évaluation des effets sur les cellules malades.

14. Procédé selon l'une quelconque des revendications 10 à 13, dans lequel l'animal est un animal malade.

15. Procédé selon l'une quelconque des revendications 10 à 14, dans lequel ledit agent est choisi dans le groupe constitué de vaccins, adjuvants, candidats immunothérapeutiques, cosmétiques, médicaments, agents biologiques et composés chimiques.

16. Procédé selon la revendication 15, dans lequel ledit agent est un vaccin.
